(19) Europäisches Patentamt
European Patent Office
Office européen des brevets

(11) **EP 3 233 056 B1**

(12) **EUROPEAN PATENT SPECIFICATION**

(45) Date of publication and mention of the grant of the patent:
**15.11.2023 Bulletin 2023/46**

(21) Application number: **15820019.6**

(22) Date of filing: **15.12.2015**

(51) International Patent Classification (IPC):
***A61K 9/00*** (2006.01) ***A61K 9/16*** (2006.01)
***A61K 9/19*** (2006.01) ***A61K 31/506*** (2006.01)
***A61K 31/404*** (2006.01) ***A61P 27/02*** (2006.01)
***A61P 27/06*** (2006.01) ***A61P 25/00*** (2006.01)
***A61P 25/02*** (2006.01) ***A61P 43/00*** (2006.01)

(52) Cooperative Patent Classification (CPC):
**A61K 31/404; A61K 9/0051; A61K 9/1647; A61K 9/19; A61K 31/506; A61P 25/00; A61P 25/02; A61P 27/02; A61P 27/06; A61P 43/00**

(86) International application number:
**PCT/US2015/065894**

(87) International publication number:
**WO 2016/100392 (23.06.2016 Gazette 2016/25)**

(54) **SUNITINIB FORMULATIONS AND METHODS FOR USE THEREOF IN TREATMENT OF OCULAR DISORDERS**

SUNITINIB-FORMULIERUNGEN UND VERFAHREN ZUR VERWENDUNG DAVON BEI DER BEHANDLUNG VON AUGENERKRANKUNGEN

FORMULATIONS DE SUNITINIB ET LEURS PROCÉDÉS D'UTILISATION DANS LE TRAITEMENT DE TROUBLES OCULAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.12.2014 US 201462092118 P**
**27.03.2015 US 201562139306 P**

(43) Date of publication of application:
**25.10.2017 Bulletin 2017/43**

(73) Proprietor: **The Johns Hopkins University**
**Baltimore, MD 21218 (US)**

(72) Inventors:
- **FU, Jie**
  **Baltimore, MD 21239 (US)**
- **HANES, Justin**
  **Baltimore, MD 21212 (US)**
- **KAYS, Joshua**
  **Allston, Maryland 02134 (US)**
- **YU, Yun**
  **Nottingham, Maryland 21236 (US)**
- **YANG, Ming**
  **Towson, MD 21286 (US)**
- **CLELAND, Jeffrey**
  **San Carlos, CA 94070 (US)**
- **STARK, Walter, J.**
  **Longboat Key, FL 34228 (US)**
- **XU, Qingguo**
  **Baltimore, MD 21210 (US)**
- **YANG, Jin**
  **Shanghai (CN)**

(74) Representative: **Bassil, Nicholas Charles et al**
**Kilburn & Strode LLP**
**Lacon London**
**84 Theobalds Road**
**London WC1X 8NL (GB)**

(56) References cited:
**WO-A2-2013/177367**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- RAMAZANI F ET AL: "Sunitinib microspheres based on [PDLLA-PEG-PDLLA]-b-PLLA multi-block copolymers for ocular drug delivery", EUROPEAN JOURNAL OF PHARMACEUTICS AND BIOPHARMACEUTICS, vol. 95, 19 February 2015 (2015-02-19), pages 368-377, XP029296806, ISSN: 0939-6411, DOI: 10.1016/J.EJPB.2015.02.011
- LI M ET AL: "Microencapsulation by solvent evaporation: State of the art for process engineering approaches", INTERNATIONAL JOURNAL OF PHARMACEUTICS, ELSEVIER BV, NL, vol. 363, no. 1-2, 3 November 2008 (2008-11-03), pages 26-39, XP025431290, ISSN: 0378-5173, DOI: 10.1016/J.IJPHARM.2008.07.018 [retrieved on 2008-07-29]
- KATRIN FUCHS ET AL: "Sunitinib-eluting beads for chemoembolization: Methods for in vitro evaluation of drug release", INTERNATIONAL JOURNAL OF PHARMACEUTICS, vol. 482, no. 1-2, 18 November 2014 (2014-11-18), pages 68-74, XP055252116, NL ISSN: 0378-5173, DOI: 10.1016/j.ijpharm.2014.11.041
- ZIMING ZHAO ET AL: "Preparation and characterization of sunitinib-loaded microspheres for arterial embolization", JOURNAL OF CHINESE PHARMACEUTICAL SCIENCES, vol. 23, no. 8, 20 August 2014 (2014-08-20), XP055252257, Beijing , CN ISSN: 1003-1057, DOI: 10.5246/jcps.2014.08.072
- HERRERO-VANRELL ROCÍO ET AL: "The potential of using biodegradable microspheres in retinal diseases and other intraocular pathologies", PROGRESS IN RETINAL AND EYE RESEARCH, OXFORD, GB, vol. 42, 10 May 2014 (2014-05-10), pages 27-43, XP029046400, ISSN: 1350-9462, DOI: 10.1016/J.PRETEYERES.2014.04.002

## Description

### CROSS REFERENCE TO RELATED APPLICATIONS

This application claims priority to and benefit of U.S. Provisional Application No. 62/092,118 "Controlled Release Sunitinib Formulations" filed on December 15, 2014, and U.S. Provisional Application No. 62/139,306 "Method Of Prevention Of Corneal Neovascularization" filed March 27, 2015.

### FIELD OF THE INVENTION

The present invention relates to formulations of sunitinib and pharmaceutically acceptable salts and methods of use thereof, especially for use in the treatment of ocular diseases and disorders.

### BACKGROUND OF THE INVENTION

Sunitinib (marketed in the form of the (-)-malic acid salt as SUTENT® by Pfizer, and previously known as SU11248) is an oral, small-molecule, multi-targeted receptor tyrosine kinase (RTK) inhibitor that was approved by the FDA for the treatment of renal cell carcinoma (RCC) and imatinib-resistant gastrointestinal stromal tumor (GIST) on January 26, 2006. Sunitinib was the first cancer drug simultaneously approved for two different indications.

Sunitinib inhibits cellular signaling by targeting multiple receptor tyrosine kinases (RTKs). These include all receptors for platelet-derived growth factor (PDGF-Rs) and vascular endothelial growth factor receptors (VEGFRs), which play a role in both tumor angiogenesis and tumor cell proliferation. The simultaneous inhibition of these targets leads to both reduced tumor vascularization and cancer cell death, and, ultimately, tumor shrinkage.

WO 2013/177367 describes process in which sunitinib is encapsulated in microspheres by means of an emulsion process.

Li et al (Int. J. Pharm., 363, 26-39 (2008)) describes microencapsulation by solvent evaporation with respect to process engineering approaches.

Fuchs et al (Int. J. Pharm., 482, 68-74 (2014)) describes Sunitinib-eluting beads for chemoembolization and methods for in vitro evaluation of drug release

Zhao et al (J. Chinese Pharm. Sci., 23, 1003-1057 (2014) describes preparation and characterization of sunitinib-loaded microspheres for arterial embolization.

Herrero-Vanrell et al (Progress in Retinal and Eye Res., 42, 27-43 (2014)) reports studies on biodegradable microspheres in retinal diseases and other intraocular pathologies.

Ramazani et al (European Journal of Pharmaceutics and Biopharmaceutics, 95, 368-377 (2015)) describes sunitinib microspheres based on [PDLLA-PEG-PDLLA]-b-PLLA multi-block copolymers for ocular drug delivery.

It would be advantageous to provide formulations that could deliver sunitinib or pharmaceutically acceptable salt in a controlled fashion, over a prolonged period of time. This has proven difficult, however, due to poor solubility of the drug in pharmaceutical excipients, limiting drug loading, and leading to instability.

It is therefore an object of the invention to provide formulations of sunitinib or pharmaceutically acceptable salt with improved duration, stability, safety, and efficacy.

It is a further object of the invention to provide methods for encapsulation or incorporation into polymeric matrices, including nano- and micro-particles, with increased loading.

It is still another object of the invention to provide improved dosage formulations, prolonged pharmacokinetics, and methods of use thereof.

### SUMMARY OF THE INVENTION

The present invention is defined in the appended claims.

The present invention provides polymeric microparticles comprising sunitinib or a pharmaceutically acceptable salt thereof encapsulated or dispersed in a blend of (a) polylactic acid and/or a copolymer of lactic acid and glycolic acid and (b) polylactic acid conjugated to a polyalkylene oxide and/or a copolymer of lactic acid and glycolic acid conjugated to a polyalkylene oxide, wherein the sunitinib or a pharmaceutically acceptable salt thereof is present in a weight loading of greater than 5% and wherein the microparticles provide sustained release of sunitinib or the pharmaceutically acceptable salt thereof.

The present invention also provides a pharmaceutical composition for ocular delivery comprising an effective amount of the polymeric microparticles of the invention in a pharmaceutically acceptable carrier.

Also disclosed herein are methods for increasing the encapsulation or incorporation of sunitinib or pharmaceutically acceptable salt into polymeric matrices have been developed. The resulting formulations provide for more sustained

controlled release of sunitinib or salt for treatment of cancer, inhibition of angiogenesis, ocular diseases, and other applications. Increased loading is achieved using an alkaline solvent system, and/or by increasing the viscosity or concentration of the polymer solution, as described in more detail below

The polymeric sunitinib drug formulation may be prepared by: (i) dissolving or dispersing sunitinib or its salt in an organic solvent optionally with an alkaline agent; (ii) mixing the solution/dispersion of step (i) with a polymer solution that has a viscosity of at least about 300 cPs (or perhaps at least about 350, 400, 500, 600, 700 or 800 or more cPs); (iii) mixing the drug polymer solution/dispersion of step (ii) with an aqueous non-acidic or alkaline solution (for example at least approximately a pH of 7, 8, or 9 and typically not higher than about 10) optionally with a surfactant or emulsifier, to form a solvent-laden sunitinib encapsulated microparticle, (iv) isolating the microparticles. When sunitinib malate or another pharmaceutically acceptable salt of sunitinib is used, it has been found that it may be useful to include the alkaline agent in the organic solvent. However, when sunitinib free base is used, then it has been found that adding an acid to the organic solvent can improve drug loading of the microparticle. Examples demonstrate that polyesters such as PLGA, PEG-PLGA( PLA) and PEG-PLGA/PLGA blend microparticles display sustained release of sunitinib or pharmaceutically acceptable salt. Polymer microparticles composed of PLGA and PEG covalently conjugated to PLGA ($M_w$ 45 kDa) (PLGA45k-PEG5k) loaded with sunitinib malate were prepared using a single emulsion solvent evaporation method. Loading improvement was achieved by increasing the alkalinity of sunitinib malate in solution, up to 16.1 % with PEG-PLGA, which could be further increased by adding DMF, compared to only 1% with no alkaline added. Sunitinib malate loading was further increased by increasing the pH of the aqueous solution as well as the polymer solution. Still further significant increases in sunitinib malate loading in the microparticles was achieved by increasing polymer concentration or viscosity.

The polymer drug composition provided herein can be used to form implants (e.g., rods, discs, wafers, etc.), nanoparticles, or microparticles with improved properties for controlled delivery of drugs. Pharmaceutical compositions containing implants (e.g., rods, discs, wafers, etc.), nanoparticles, microparticles, or combinations thereof for the controlled release of the sunitinib or pharmaceutically acceptable salt thereof can be prepared by combining the drug in the matrix with one or more pharmaceutically acceptable excipients. The nanoparticles, microparticles, or combination thereof can be formed from one or more drugs, or blends of drugs with one or more polymers.

The pharmaceutical compositions can be administered to treat or prevent a disease or disorder in or on the eye of a patient associated with neovascularization, such as comeal neovascularization and wet or dry age-related macular degeneration (AMD).

Illustrative examples confirm in animal models that the formulations of sunitinib or its pharmaceutically acceptable salt are efficacious in treating comeal neovascularization, choroidal vascularization characteristic of AMD and in preventing optic nerve damage due to elevated intraocular pressure.

## BRIEF DESCRIPTION OF THE DRAWINGS

Figure 1 is a graph of percent encapsulation efficiency as a function of polymer concentration (mg/ml).

Figure 2A is a graph of percent cumulative release of sunitinib malate at 37°C from various polymer microparticles over time (days).

Figure 2B is a graph showing that increasing polymer concentration improves encapsulation efficiency of sunitinib malate, plotting percent encapsulation efficiency against polymer concentration (mg/mL).

Figure 3 is a graph of the *in vitro* drug release profile of sunitinib malatee MS (microsphere).

Figure 4 is a graph of the retention curve of sunitinib malate free drug and sunitinib-malate MS.

Figures 5A and 5B are graphs of quantitative analysis of comeal neovascularization (vessel length, Figure 5A and NV area, Figure 5B) of the corneas at POD 5 , POD 7 and POD 14 with the treatment with SC injection of sunitinib malate MS, sunitinib malate free drug and Placebo MS.

Figures 6A-6M are bar graphs of RT-PCR analysis revealing the strong suppression of the expression levels of drug target genes by sunitinib malate MS compared to sunitinib malate and Placebo MS on POD 7.

Figures 7A-7D are graphs showing that sunitinib malate microparticles suppress NV in mouse CNV model for at least 9 weeks following intravitreal injection into to normal C57Bl/6 mice. Immediately after or 2, 4, or 8 weeks later, mice (n=5) were subjected to laser disruption of Bruch's membrane, and one week later the size of the CNV lesions was quantitated. Figure 7A, one week; Figure 7B, three weeks; Figure 7C, five weeks; Figure 7D, nine weeks. P<0.05 for all treated groups compared to controls.

## DETAILED DESCRIPTION OF THE INVENTION

### I. Definitions

"Active Agent," as used herein, refers to a physiologically or pharmacologically active substance that acts locally and/or systemically in the body. An active agent is a substance that is administered to a patient for the treatment (*e.g.,* therapeutic agent), prevention (*e.g.,* prophylactic agent), or diagnosis (*e.g.,* diagnostic agent) of a disease or disorder. "Ophthalmic Drug" or "Ophthalmic Active Agent", as used herein, refers to an agent that is administered to a patient to alleviate, delay onset of, or prevent one or more symptoms of a disease or disorder of the eye, or diagnostic agent useful for imaging or otherwise assessing the eye.

"Effective amount" or "therapeutically effective amount," as used herein, refers to an amount of drug effective to alleviate, delay onset of, or prevent one or more symptoms, particularly of cancer or a disease or disorder of the eye. In the case of age-related macular degeneration, the effective amount of the drug delays, reduces, or prevents vision loss in a patient.

As used herein, the term "alkaline" refers to a compound capable of accepting an acidic proton or otherwise raising the pH of the composition.

"Biocompatible" and "biologically compatible," as used herein, generally refer to materials that are, along with any metabolites or degradation products thereof, generally non-toxic to the recipient, and do not cause any significant adverse effects to the recipient. Generally speaking, biocompatible materials are materials which do not elicit a significant inflammatory or immune response when administered to a patient.

"Biodegradable Polymer," as used herein, generally refers to a polymer that will degrade or erode by enzymatic action and/or hydrolysis under physiologic conditions to smaller units or chemical species that are capable of being metabolized, eliminated, or excreted by the subject. The degradation time is a function of polymer composition, morphology, such as porosity, particle dimensions, and environment.

"Hydrophilic," as used herein, refers to the property of having affinity for water. For example, hydrophilic polymers (or hydrophilic polymers) are polymers (or polymers) which are primarily soluble in aqueous solutions and/or have a tendency to absorb water. In general, the more hydrophilic a polymer is, the more that polymer tends to dissolve in, mix with, or be wetted by water.

"Hydrophobic," as used herein, refers to the property of lacking affinity for, or even repelling water. For example, the more hydrophobic a polymer (or polymer), the more that polymer (or polymer) tends to not dissolve in, not mix with, or not be wetted by water.

Hydrophilicity and hydrophobicity can be spoken of in relative terms, such as, but not limited to, a spectrum of hydrophilicity/hydrophobicity within a group of polymers or polymers. In some embodiments wherein two or more polymers are being discussed, the term "hydrophobic polymer" can be defined based on the polymer's relative hydrophobicity when compared to another, more hydrophilic polymer.

"Nanoparticle," as used herein, generally refers to a particle having a diameter, such as an average diameter, from about 10 nm up to but not including about 1 micron, for example, from 100 nm to about 1 micron. The particles can have any shape. Nanoparticles having a spherical shape are generally referred to as "nanospheres".

"Microparticle," as used herein, generally refers to a particle having a diameter, such as an average diameter, from about 1 micron to about 100 microns, for example, from about 1 micron to about 50 microns, more for example, from about 1 to about 30 microns. The microparticles can have any shape. Microparticles having a spherical shape are generally referred to as "microspheres" ("MS").

"Molecular weight," as used herein, generally refers to the relative average chain length of the bulk polymer, unless otherwise specified. In practice, molecular weight can be estimated or characterized using various methods including gel permeation chromatography (GPC) or capillary viscometry. GPC molecular weights are reported as the weight-average molecular weight (Mw) as opposed to the number-average molecular weight (Mn). Capillary viscometry provides estimates of molecular weight as the inherent viscosity determined from a dilute polymer solution using a particular set of concentration, temperature, and solvent conditions.

"Mean particle size," as used herein, generally refers to the statistical mean particle size (diameter) of the particles in a population of particles. The diameter of an essentially spherical particle may refer to the physical or hydrodynamic diameter. The diameter of a non-spherical particle may refer preferentially to the hydrodynamic diameter. As used herein, the diameter of a non-spherical particle may refer to the largest linear distance between two points on the surface of the particle. Mean particle size can be measured using methods known in the art, such as dynamic light scattering.

"Monodisperse" and "homogeneous size distribution" are used interchangeably herein and describe a population of nanoparticles or microparticles where all of the particles are the same or nearly the same size. As used herein, a monodisperse distribution refers to particle distributions in which 90% or more of the distribution lies within 15% of the median particle size, more for example, within 10% of the median particle size, most for example, within 5% of the

median particle size.

"Pharmaceutically Acceptable," as used herein, refers to compounds, carriers, excipients, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

"Implant," as generally used herein, refers to a polymeric device or element that is structured, sized, or otherwise configured to be implanted, for example, by injection or surgical implantation, in a specific region of the body so as to provide therapeutic benefit by releasing one or more active agents over an extended period of time at the site of implantation. For example, intraocular implants are polymeric devices or elements that are structured, sized, or otherwise configured to be placed in the eye, for example, by injection or surgical implantation, and to treat one or more diseases or disorders of the eye by releasing one or more drugs over an extended period. Intraocular implants are generally biocompatible with physiological conditions of an eye and do not cause adverse side effects. Generally, intraocular implants may be placed in an eye without disrupting vision of the eye.

## II. *Compositions*

### *A. Sunitinib*

Sunitinib is a compound of formula (1):

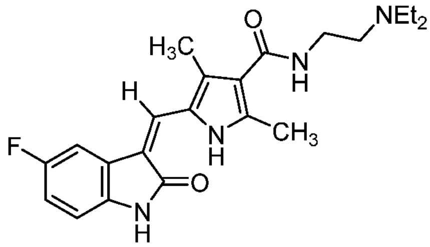

Sunitinib malate is the (-)-malic acid salt of sunitinib, which is sold as Sutent:

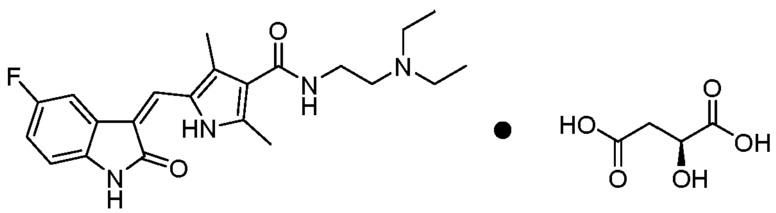

The following definitions are used herein:

"Alkyl" refers to a saturated aliphatic hydrocarbon radical including straight chain and branched chain groups of 1 to 20 carbon atoms (whenever a numerical range; e.g. "1-20", is stated herein, it means that the group, in this case the alkyl group, may contain 1 carbon atom, 2 carbon atoms, 3 carbon atoms, etc. up to and including 20 carbon atoms). Alkyl groups containing from 1 to 4 carbon atoms are referred to as lower alkyl groups. When the lower alkyl groups lack substituents, they are referred to as unsubstituted lower alkyl groups. More for example, an alkyl group is a medium size alkyl having 1 to 10 carbon atoms e.g., methyl, ethyl, propyl, 2-propyl, n-butyl, iso-butyl, tert-butyl, and pentyl. Most for example, it is a lower alkyl having 1 to 4 carbon atoms e.g., methyl, ethyl, propyl, 2-propyl, n-butyl, iso-butyl, or tert-butyl. The alkyl group may be substituted or unsubstituted. When substituted, the substituent group(s) is for example, one or more, more for example, one to three, even more for example, one or two substituent(s) independently selected from the group consisting of halo, hydroxy, unsubstituted lower alkoxy, aryl optionally substituted with one or more groups, for example, one, two or three groups which are independently of each other halo, hydroxy, unsubstituted lower alkyl or unsubstituted lower alkoxy groups, aryloxy optionally substituted with one or more groups, for example, one, two or three groups which are independently of each other halo, hydroxy, unsubstituted lower alkyl or unsubstituted lower alkoxy groups, 6-member heteroaryl having from 1 to 3 nitrogen atoms in the ring, the carbons in the ring being optionally substituted with one or more groups, for example, one, two or three groups which are independently of each other halo, hydroxy, unsubstituted lower alkyl or unsubstituted lower alkoxy groups, 5-member heteroaryl having from 1 to 3 heteroatoms selected from the group consisting of nitrogen, oxygen and sulfur, the carbon and the nitrogen atoms in the group being optionally substituted with one or more groups, for example, one, two or three groups which are

independently of each other halo, hydroxy, unsubstituted lower alkyl or unsubstituted lower alkoxy groups, 5- or 6-member heteroalicyclic group having from 1 to 3 heteroatoms selected from the group consisting of nitrogen, oxygen and sulfur, the carbon and nitrogen (if present) atoms in the group being optionally substituted with one or more groups, for example, one, two or three groups which are independently of each other halo, hydroxy, unsubstituted lower alkyl or unsubstituted lower alkoxy groups, mercapto, (unsubstituted lower alkyl)thio, arylthio optionally substituted with one or more groups, for example, one, two or three groups which are independently of each other halo, hydroxy, unsubstituted lower alkyl or unsubstituted lower alkoxy groups, cyano, acyl, thioacyl, O-carbamyl, N-carbamyl, O-thiocarbamyl, N-thiocarbamyl, C-amido, N-amido, nitro, N-sulfonamido, S-sulfonamido, $R^{18}S(O)$-, $R^{18}S(O)_2$-, -$C(O)OR^{18}$, $R^{18}C(O)O$-, and -$NR^{18}R^{19}$, wherein $R^{18}$ and $R^{19}$ are independently selected from the group consisting of hydrogen, unsubstituted lower alkyl, trihalomethyl, unsubstituted ($C_3$-$C_6$)cycloalkyl, unsubstituted lower alkenyl, unsubstituted lower alkynyl and aryl optionally substituted with one or more, groups, for example, one, two or three groups which are independently of each other halo, hydroxy, unsubstituted lower alkyl or unsubstituted lower alkoxy groups.

In one embodiment, the alkyl group is substituted with one or two substituents independently selected from the group consisting of hydroxy, 5- or 6-member heteroalicyclic group having from 1 to 3 heteroatoms selected from the group consisting of nitrogen, oxygen and sulfur, the carbon and nitrogen (if present) atoms in the group being optionally substituted with one or more groups, for example, one, two or three groups which are independently of each other halo, hydroxy, unsubstituted lower alkyl or unsubstituted lower alkoxy groups, 5-member heteroaryl having from 1 to 3 heteroatoms selected from the group consisting of nitrogen, oxygen and sulfur, the carbon and the nitrogen atoms in the group being optionally substituted with one or more groups, for example, one, two or three groups which are independently of each other halo, hydroxy, unsubstituted lower alkyl or unsubstituted lower alkoxy groups, 6-member heteroaryl having from 1 to 3 nitrogen atoms in the ring, the carbons in the ring being optionally substituted with one or more groups, for example, one, two or three groups which are independently of each other halo, hydroxy, unsubstituted lower alkyl or unsubstituted lower alkoxy groups, or -$NR^{18}R^{19}$, wherein $R^{18}$ and $R^{19}$ are independently selected from the group consisting of hydrogen, unsubstituted lower alkyl. In some embodiments, for example, the alkyl group is substituted with one or two substituents which are independently of each other hydroxy, dimethylamino, ethylamino, diethylamino, dipropylamino, pyrrolidino, piperidino, morpholino, piperazino, 4-lower alkylpiperazino, phenyl, imidazolyl, pyridinyl, pyridazinyl, pyrimidinyl, oxazolyl, and triazinyl.

"Cycloalkyl" refers to a 3 to 8 member all-carbon monocyclic ring, an all-carbon 5-member/6-member or 6-member/6-member fused bicyclic ring or a multicyclic fused ring (a "fused" ring system means that each ring in the system shares an adjacent pair of carbon atoms with each other ring in the system) group wherein one or more of the rings may contain one or more double bonds but none of the rings has a completely conjugated pi-electron system. Examples of cycloalkyl groups are cyclopropane, cyclobutane, cyclopentane, cyclopentene, cyclohexane, cyclohexadiene, adamantane, cycloheptane, and cycloheptatriene. A cycloalkyl group may be substituted or unsubstituted. When substituted, the substituent group(s) is for example, one or more, for example one or two substituents, independently selected from the group consisting of unsubstituted lower alkyl, trihaloalkyl, halo, hydroxy, unsubstituted lower alkoxy, aryl optionally substituted with one or more, for example, one or two groups independently of each other halo, hydroxy, unsubstituted lower alkyl or unsubstituted lower alkoxy groups, aryloxy optionally substituted with one or more, for example, one or two groups independently of each other halo, hydroxy, unsubstituted lower alkyl or unsubstituted lower alkoxy groups, 6-member heteroaryl having from 1 to 3 nitrogen atoms in the ring, the carbons in the ring being optionally substituted with one or more, for example, one or two groups independently of each other halo, hydroxy, unsubstituted lower alkyl or unsubstituted lower alkoxy groups, 5-member heteroaryl having from 1 to 3 heteroatoms selected from the group consisting of nitrogen, oxygen and sulfur, the carbon and nitrogen atoms of the group being optionally substituted with one or more, for example, one or two groups independently of each other halo, hydroxy, unsubstituted lower alkyl or unsubstituted lower alkoxy groups, 5- or 6-member heteroalicyclic group having from 1 to 3 heteroatoms selected from the group consisting of nitrogen, oxygen and sulfur, the carbon and nitrogen (if present) atoms in the group being optionally substituted with one or more, for example, one or two groups independently of each other halo, hydroxy, unsubstituted lower alkyl or unsubstituted lower alkoxy groups, mercapto,(unsubstituted lower alkyl)thio, arylthio optionally substituted with one or more, for example, one or two groups independently of each other halo, hydroxy, unsubstituted lower alkyl or unsubstituted lower alkoxy groups, cyano, acyl, thioacyl, O-carbamyl, N-carbamyl, O-thiocarbamyl, N-thiocarbamyl, C-amido, N-amido, nitro, N-sulfonamido, S-sulfonamido, $R^{18}S(O)$-, $R^{18}S(O)_2$-, -$C(O)OR^{18}$, $R^{18}C(O)O$-, and -$NR^{18}R^{19}$ are as defined above.

"Alkenyl" refers to a lower alkyl group, as defined herein, consisting of at least two carbon atoms and at least one carbon-carbon double bond. Representative examples include, but are not limited to, ethenyl, 1-propenyl, 2-propenyl, and 1-, 2-, or 3-butenyl.

"Alkynyl" refers to a lower alkyl group, as defined herein, consisting of at least two carbon atoms and at least one carbon-carbon triple bond. Representative examples include, but are not limited to, ethynyl, 1-propynyl, 2-propynyl, and 1-, 2-, or 3-butynyl.

"Aryl" refers to an all-carbon monocyclic or fused-ring polycyclic (i.e., rings which share adjacent pairs of carbon

atoms) groups of 1 to 12 carbon atoms having a completely conjugated pi-electron system. Examples, without limitation, of aryl groups are phenyl, naphthalenyl and anthracenyl. The aryl group may be substituted or unsubstituted. When substituted, the substituted group(s) is , for example, one or more, for example, one, two or three, , independently selected from the group consisting of unsubstituted lower alkyl, trihaloalkyl, halo, hydroxy, unsubstituted lower alkoxy, mercapto,(unsubstituted lower alkyl)thio, cyano, acyl, thioacyl, O-carbamyl, N-carbamyl, O-thiocarbamyl, N-thiocarbamyl, C-amido, N-amido, nitro, N-sulfonamido, S-sulfonamido, $R^{18}S(O)$-, $R^{18}S(O)_2$-, -C(O)$OR^{18}$, $R^{18}C(O)O$-, and -$NR^{18}R^{19}$, with $R^{18}$ and $R^{19}$ as defined above. For example, the aryl group is optionally substituted with one or two substituents independently selected from halo, unsubstituted lower alkyl, trihaloalkyl, hydroxy, mercapto, cyano, N-amido, mono or dialkylamino, carboxy, or N-sulfonamido.

"Heteroaryl" refers to a monocyclic or fused ring (i.e., rings which share an adjacent pair of atoms) group of 5 to 12 ring atoms containing one, two, or three ring heteroatoms selected from N, O, or S, the remaining ring atoms being C, and, in addition, having a completely conjugated pi-electron system. Examples, without limitation, of unsubstituted heteroaryl groups are pyrrole, furan, thiophene, imidazole, oxazole, thiazole, pyrazole, pyridine, pyrimidine, quinoline, isoquinoline, purine and carbazole. The heteroaryl group may be substituted or unsubstituted. When substituted, the substituted group(s) is , for example, one, two, or three, independently selected from the group consisting of unsubstituted lower alkyl, trihaloalkyl, halo, hydroxy, unsubstituted lower alkoxy, mercapto,(unsubstituted lower alkyl)thio, cyano, acyl, thioacyl, O-carbamyl, N-carbamyl, O-thiocarbamyl, N-thiocarbamyl, C-amido, N-amido, nitro, N-sulfonamido, S-sulfonamido, $R^{18}S(O)$-, $R^{18}O)_2$-, -C(O)$OR^{18}$, $R^{18}C(O)O$-, and -$NR^{18}R^{19}$, with $R^{18}$ and $R^{19}$ as defined above. For example, the heteroaryl group is optionally substituted with one or two substituents independently selected from halo, unsubstituted lower alkyl, trihaloalkyl, hydroxy, mercapto, cyano, N-amido, mono or dialkylamino, carboxy, or N-sulfonamido.

"Heteroalicyclic" refers to a monocyclic or fused ring group having in the ring(s) of 5 to 9 ring atoms in which one or two ring atoms are heteroatoms selected from N, O, or $S(O)_n$ (where n is an integer from 0 to 2), the remaining ring atoms being C. The rings may also have one or more double bonds. However, the rings do not have a completely conjugated pi-electron system. Examples, without limitation, of unsubstituted heteroalicyclic groups are pyrrolidino, piperidino, piperazino, morpholino, thiomorpholino, and homopiperazino. The heteroalicyclic ring may be substituted or unsubstituted. When substituted, the substituted group(s) is one or more, for example one, two or three, independently selected from the group consisting of unsubstituted lower alkyl, trihaloalkyl, halo, hydroxy, unsubstituted lower alkoxy, mercapto,(unsubstituted lower alkyl)thio, cyano, acyl, thioacyl, O-carbamyl, N-carbamyl, O-thiocarbamyl, N-thiocarbamyl, C-amido, N-amido, nitro, N-sulfonamido, S-sulfonamido, $R^{18}S(O)$-, $R^{18}S(O)_2$-, -C(O)$OR^{18}$, $R^{18}C(O)O$-, and -$NR^{18}R^{19}$, with $R^{18}$ and $R^{19}$ as defined above. For example, the heteroalicyclic group is optionally substituted with one or two substituents independently selected from halo, unsubstituted lower alkyl, trihaloalkyl, hydroxy, mercapto, cyano, N-amido, mono or dialkylamino, carboxy, or N-sulfonamido.

For example, the heteroalicyclic group is optionally substituted with one or two substituents independently selected from halo, unsubstituted lower alkyl, trihaloalkyl, hydroxy, mercapto, cyano, N-amido, mono or dialkylamino, carboxy, or N-sulfonamido.

"Heterocycle" means a saturated cyclic radical of 3 to 8 ring atoms in which one or two ring atoms are heteroatoms selected from N, O, or $S(O)_n$ (where n is an integer from 0 to 2), the remaining ring atoms being C, where one or two C atoms may optionally be replaced by a carbonyl group. The heterocyclyl ring may be optionally substituted independently with one, two, or three substituents selected from optionally substituted lower alkyl (substituted with 1 or 2 substituents independently selected from carboxy or ester), haloalkyl, cyanoalkyl, halo, nitro, cyano, hydroxy, alkoxy, amino, monoalkylamino, dialkylamino, aralkyl, heteroaralkyl, —COR (where R is alkyl) or -COOR where R is (hydrogen or alkyl). More specifically the term heterocyclyl includes, but is not limited to, tetrahydropyranyl, 2,2-dimethyl-1,3-dioxolane, piperidino, N-methylpiperidin-3-yl, piperazino, N-methylpyrrolidin 3-yl, 3-pyrrolidino, morpholino, thiomorpholino, thiomorpholino-1-oxide, thiomorpholino 1,1-dioxide, 4-ethyloxycarbonylpiperazino, 3-oxopiperazino, 2-imidazolidone, 2-pyrrolidinone, 2-oxohomopiperazino, tetrahydropyrimidin-2-one, and the derivatives thereof. For example, the heterocycle group is optionally substituted with one or two substituents independently selected from halo, unsubstituted lower alkyl, lower alkyl substituted with carboxy, ester, hydroxy, mono or dialkylamino.

"Hydroxy" refers to an -OH group.

"Alkoxy" refers to both an -O-(unsubstituted alkyl) and an -O-(unsubstituted cycloalkyl) group. Representative examples include, but are not limited to, e.g., methoxy, ethoxy, propoxy, butoxy, cyclopropyloxy, cyclobutyloxy, cyclopentyloxy, and cyclohexyloxy.

"Aryloxy" refers to both an -O-aryl and an -O-heteroaryl group, as defined herein. Representative examples include, but are not limited to, phenoxy, pyridinyloxy, furanyloxy, thienyloxy, pyrimidinyloxy, pyrazinyloxy, and derivatives thereof.

"Mercapto" refers to an -SH group.

"Alkylthio" refers to both an -S-(unsubstituted alkyl) and an -S-(unsubstituted cycloalkyl) group. Representative examples include, but are not limited to, e.g., methylthio, ethylthio, propylthio, butylthio, cyclopropylthio, cyclobutylthio, cyclopentylthio, and cyclohexylthio.

"Arylthio" refers to both an -S-aryl and an -S-heteroaryl group, as defined herein. Representative examples include, but are not limited to, phenylthio, pyridinylthio, furanylthio, thientylthio, pyrimidinylthio, and derivatives thereof.

"Acyl" refers to a -C(O)-R" group, where R" is selected from the group consisting of hydrogen, unsubstituted lower alkyl, trihalomethyl, unsubstituted cycloalkyl, aryl optionally substituted with one or more, for example, one, two, or three substituents selected from the group consisting of unsubstituted lower alkyl, trihalomethyl, unsubstituted lower alkoxy, halo and $-NR^{18}R^{19}$ groups, heteroaryl (bonded through a ring carbon) optionally substituted with one or more, for example, one, two, or three substitutents selected from the group consisting of unsubstituted lower alkyl, trihaloalkyl, unsubstituted lower alkoxy, halo and $-NR^{18}R^{19}$ groups and heteroalicyclic (bonded through a ring carbon) optionally substituted with one or more, for example, one, two, or three substituents selected from the group consisting of unsubstituted lower alkyl, trihaloalkyl, unsubstituted lower alkoxy, halo and $-NR^{18}R^{19}$ groups. Representative acy groups include, but are not limited to, acetyl, trifluoroacetyl, and benzoyl.

"Aldehyde" refers to an acyl group in which R" is hydrogen.

"Thioacyl" refers to a -C(S)-R" group, with R" as defined herein.

"Ester" refers to a -C(O)O-R" group with R" as defined herein except that R" cannot be hydrogen.

"Acetyl" group refers to a $-C(O)CH_3$ group.

"Halo" group refers to fluorine, chlorine, bromine or iodine, for example fluorine or chlorine.

"Trihalomethyl" group refers to a $-CX_3$ group wherein X is a halo group as defined herein.

"Trihalomethanesulfonyl" group refers to a $X_3CS(=O)_2$- groups with X as defined above.

"Cyano" refers to a -C≡N group.

"Methylenedioxy" refers to a $-OCH_2O-$ group where the two oxygen atoms are bonded to adjacent carbon atoms.

"Ethylenedioxy" group refers to a $-OCH_2CH_2O-$ where the two oxygen atoms are bonded to adjacent carbon atoms.

"S-sulfonamido" refers to a $-S(O)_2NR^{18}R^{19}$ group, with $R^{18}$ and $R^{19}$ as defined herein. "N-sulfonamido" refers to a $-NR^{18}S(O)_2R^{19}$ group, with $R^{18}$ and $R^{19}$ as defined herein.

"O-carbamyl" group refers to a $-OC(O)NR^{18}R^{19}$ group with $R^{18}$ and $R^{19}$ as defined herein. "N-carbamyl" refers to an $R^{18}OC(O)NR^{19}$- group, with $R^{18}$ and $R^{19}$ as defined herein.

"O-thiocarbamyl" refers to a $-OC(S)NR^{18}R^{19}$ group with $R^{18}$ and $R^{19}$ as defined herein. "N-thiocarbamyl" refers to a $R^{18}OC(S)NR^{19}$- group, with $R^{18}$ and $R^{19}$ as defined herein.

"Amino" refers to an $-NR^{18}R^{19}$ group, wherein $R^{18}$ and $R^{19}$ are both hydrogen.

"C-amido" refers to a $-C(O)NR^{18}R^{19}$ group with $R^{18}$ and $R^{19}$ as defined herein. "N-amido" refers to a $R^{18}C(O)NR^{19}$- group, with $R^{18}$ and $R^{19}$ as defined herein.

"Nitro" refers to a $-NO_2$ group.

"Haloalkyl" means an unsubstituted alkyl, for example, unsubstituted lower alkyl as defined above that is substituted with one or more same or different halo atoms, e.g., $-CH_2Cl$, $-CF_3$, $-CH_2CF_3$, and $-CH_2CCl_3$.

"Aralkyl" means unsubstituted alkyl, for example, unsubstituted lower alkyl as defined above which is substituted with an aryl group as defined above, e.g., $-CH_2$phenyl, $-(CH_2)_2$phenyl, $-(CH_2)_3$phenyl, $CH_3CH(CH_3)CH_2$phenyl, and derivatives thereof.

"Heteroaralkyl" group means unsubstituted alkyl, for example, unsubstituted lower alkyl as defined above, which is substituted with a heteroaryl group as defined above,

"Dialkylamino" means a radical —NRR where each R is independently an unsubstitued alkyl or unsubstituted cycloalkyl group as defined above, e.g., dimethylamino, diethylamino, (1-methylethyl)-ethylamino, cyclohexylmethylamino, and cyclopentylmethylamino.

"Cyanoalkyl" means unsubstituted alkyl, for example, unsubstituted lower alkyl as defined above, which is substituted with 1 or 2 cyano groups.

"Optional" or "optionally" means that the subsequently described event or circumstance may but need not occur, and that the description includes instances where the event or circumstance occurs and instances in which it does not. For example, "heterocycle group optionally substituted with an alkyl group" means that the alkyl may but need not be present, and the description includes situations where the heterocycle group is substituted with an alkyl group and situations where the heterocyclo group is not substituted with the alkyl group.

### B. Encapsulating Polymers

Controlled release dosage formulations for the delivery of one or more drugs in a polymeric vehicle are described herein. The polymeric matrix can be formed from non-biodegradable or biodegradable polymers; however, the polymer matrix is preferably biodegradable. The polymeric matrix can be formed into implants (e.g., rods, disks, wafers, etc.), microparticles, nanoparticles, or combinations thereof for delivery. Upon administration, the sunitinib or pharmaceutically acceptable salt is released over an extended period of time, either upon degradation of the polymer matrix, diffusion of the one or more inhibitors out of the polymer matrix, or a combination thereof. The drug can be dispersed or encapsulated

into the polymer or covalently bound to the polymer used to form the matrix. The degradation profile of the one or more polymers may be selected to influence the release rate of the active agent *in vivo.*

The polymers may be hydrophobic, hydrophilic, conjugates of hydrophilic and hydrophobic polymers (i.e., amphiphilic polymers), block co-polymers, and blends thereof.

Examples of suitable hydrophobic polymers include, but are not limited to, polyhydroxyesters such as polylactic acid, polyglycolic acid, or copolymers thereof, polycaprolactone, polyanhydrides such as polysebacic anhydride, polydioxidone, blends and copolymers of any of the above. In one embodiment, a blend of PLGA and polylactic acid (PLA) is used. Higher molecular weight polymers, having different ratio of lactic acid (LA) ( which has a longer degradation time, up to one or two years ) to glycolic acid (GA) ( which has a short degradation time, as short as a few days to a week), are used to provide release over a longer period of time. PLGA hydrophicility can be controlled by selecting the monomer ratio of LA and GA ( more hydrophilic), the PLGA end group ( ester or acid) also affects degradation. The acid end of PLGA will also degrade faster. Acid end groups of PLGA help increase the drug loading, but also change the acid value. However, with the acid value control, even with low acid in the polymer, it can still be used to achieve higher drug loading. The PLGA can be made more hydrophilic by treating the polymer with carboxyl.

The one or more hydrophilic polymers can be any hydrophilic, biocompatible, non-toxic polymer or copolymer. In certain embodiments, the one or more hydrophilic polymers contain a poly(alkylene glycol), such as polyethylene glycol (PEG). In particular embodiments, the one or more hydrophilic polymers are linear PEG chains.

Representative synthetic polymers include poly(hydroxy acid)s such as poly(lactic acid), poly(glycolic acid), and poly(lactic acid-co-glycolic acid), poly(lactide), poly(glycolide), poly(lactide-co-glycolide), polyanhydrides, polyorthoesters, polyamides, polycarbonates, polyalkylenes such as polyethylene and polypropylene, polyalkylene glycols such as poly(ethylene glycol), polyalkylene oxides such as poly(ethylene oxide), polyalkylene terephthalates such as poly(ethylene terephthalate), polyvinyl alcohols, polyvinyl ethers, polyvinyl esters, polyvinyl halides such as poly(vinyl chloride), polyvinylpyrrolidone, polysiloxanes, poly(vinyl alcohols), poly(vinyl acetate), polystyrene, polyurethanes and co-polymers thereof, celluloses such as alkyl cellulose, hydroxyalkyl celluloses, cellulose ethers, cellulose esters, nitro celluloses, methyl cellulose, ethyl cellulose, hydroxypropyl cellulose, hydroxy-propyl methyl cellulose, hydroxybutyl methyl cellulose, cellulose acetate, cellulose propionate, cellulose acetate butyrate, cellulose acetate phthalate, carboxylethyl cellulose, cellulose triacetate, and cellulose sulphate sodium salt (jointly referred to herein as "celluloses"), polymers of acrylic acid, methacrylic acid or copolymers or derivatives thereof including esters, poly(methyl methacrylate), poly(ethyl methacrylate), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate) (jointly referred to herein as "polyacrylic acids"), poly(butyric acid), poly(valeric acid), and poly(lactide-co-caprolactone), copolymers and blends thereof. As used herein, "derivatives" include polymers having substitutions, additions of chemical groups, for example, alkyl, alkylene, hydroxylations, oxidations, and other modifications routinely made by those skilled in the art.

Examples of typical natural polymers include proteins such as albumin and prolamines, for example, zein, and polysaccharides such as alginate, cellulose and polyhydroxyalkanoates, for example, polyhydroxybutyrate.

Examples of typical non-biodegradable polymers include ethylene vinyl acetate, poly(meth)acrylic acid, polyamides, copolymers and mixtures thereof.

### C. Solvents and Alkalizing Agents

Sunitinb or a pharmaceutically acceptable salt (including the (-)-malate salt) can be used to make particles as described herein. Free base is more hydrophobic, and the salt form such as malate is more hydrophilic. The drug loading can be increased by change the form of the sunitinib. For example, adding alkaline (in both organic phase and water phase) increases sunitinib malate loading. Sunitinib free base is very hydrophobic and easily crystallized. Crystallization can be avoided and better particles formed by adding acid, or controlling the pH of the water phase.

Typical solvents for forming particles are organic solvents such as methylene chloride, chloroform, carbon tetrachloride, dichloroethane, ethyl acetate and cyclohexane. Additional solvents include, but not limited to, acetone, alcohol, acetonitrile, DMSO, and DMF. Water soluble solvents and alkaline solvents help increased the sunitinib malate loading.

It was discovered that the loading of sunitinib can be increased by increasing the alkalinity of the sunitinib in solution during encapsulation. This can be achieved by selection of the solvent, adding alkalizing agents to the solvent, or including alkaline drugs with the sunitinib. Examples of compounds that can be added for this purpose include solvents or solvent additives such as dimethylacetamide (DMA), DMTA, triethylamine (TEA), aniline, ammonium, and sodium hydroxide, drugs such as Vitamin B4, caffeine, alkaloids, nicotine, the analgesic morphine, the antibacterial berberine, the anticancer compound vincristine, the antihypertension agent reserpine, the cholinomimetic galantamine, the anticholinergic agent atropine, the vasodilator vincamine, the antiarrhythmia compound quinidine, the antiasthma therapeutic ephedrine, and the antimalarial drug quinine.

Surfactants include anionic, cationic and non-ionic surfactants, such as, but not limited to, polyvinyl alcohol, F-127, lectin, fatty acids, phospholipids, polyoxyethylene sorbitan fatty acid derivatives, and castor oil.

## III. Methods of Forming Microparticles, Nanoparticles and Implants

### A. Micro and Nanoparticle Formation

Microparticle and nanoparticles can be formed using any suitable method for the formation of polymer micro- or nanoparticles known in the art. The method employed for particle formation will depend on a variety of factors, including the characteristics of the polymers present in the drug or polymer matrix, as well as the desired particle size and size distribution. The type of drug(s) being incorporated in the particles may also be a factor as some drugs are unstable in the presence of certain solvents, in certain temperature ranges, and/or in certain pH ranges.

Particles having an average particle size of between 10 nm and 1000 microns are useful in the compositions described herein. In certain embodiments, the particles have an average particle size of between 10 nm and 100 microns, for example, between about 100 nm and about 50 microns, or between about 200 nm and about 50 microns. The particles can have any shape but are generally spherical in shape.

The drug loading in the particle is significantly affected by the acid value. For example, raising the pH by addition of alkaline significantly increases the amount of sunitinib malate incorporated. Loading also can be increased by changing the water phase pH. For example, when water phase (such as PBS) pH is raised from 6.8 to 7.4. Drug loading can also be increased by increasing both polymer and drug concentration, polymer molecular weight.

The preferred aqueous pH is higher than 6 and lower than 10, more for example, between pH 6 and 8.

For example, one of the examples in Table 2 shows that for the same particle composition, there is a substantial increase of encapsulation efficiency from 36% to 84% when the aqueous pH was increased from approximately 6 to approximately 7.4. Another example in Table 2 shows that at pH 10, the morphology of many particles changed from spherical to irregular shapes and some particles formed aggregates, suggesting aqueous solution of high pH is also unfavorable for producing particles of high loading of sunitinib and high quality.

Polymer concentration and viscosity also affects encapsulation efficiency. For example, for the same formulation composition (99% PLGA 75:25 4A and 1% PLGA-PEG (PEG MW 5 Kd, PLGA MW ~ 45 Kd)) at different polymer concentrations in dichloromethane (DCM), the encapsulation efficiency increases to over 50% at 100 mg/mL polymer concentration. The dynamic viscosity of this polymer solution in DCM, prior to mixing with sunitninb malate solution in DMSO, is estimated to be around 350 cPs. The preferred minimal viscosity of polymer solution in DCM is about 350 cPs. In a preferred embodiment, the polymer concentration in DCM is 140 mg/mL, which is approximately 720 cPs by calculation. Particles made of 99% PLGA 7525 6E and 1% PLGA-PEG (PEG MW 5 Kd, PLGA MW ~ 45 Kd) have a polymer concentration in DCM of 100 mg/mL. Since PLGA 7525 6E is a polymer with higher Mw than that of PLGA 7525 4A, the polymer solution in DCM is more viscous with a dynamic viscosity of about 830 cPs.

Drug loading is also significantly affected by the method of making and the solvent used. For example, S/O/W single emotion method will yield a higher loading than O/W single emulsion method even without control the acid value.

### Release of drug

The release of drug is influenced by a variety of factors, including molecular weight of polymer, hydrophicility or hydrophobicity of the polymer, percentage of drug, method of making particles. Both sunitinib or its pharmaceutically acceptable salt can be used to make particles. Free base is more hydrophobic, and the sunitinib free base release is much slower than sunitinib malate. The release medium also effects the drug release. The release will be increased with the medium pH increase.

### Methods of Making

Common techniques for preparing microparticles and nanoparticles include, but are not limited to, solvent evaporation, solvent removal, spray drying, phase inversion, coacervation, and low temperature casting. Suitable methods of particle formulation are briefly described below. Pharmaceutically acceptable excipients, including pH modifying agents, disintegrants, preservatives, and antioxidants, can optionally be incorporated into the particles during particle formation.

In the preferred embodiment, the formulations are made by emulsion.

1. Solvent Evaporation

In this method, the drug (or polymer matrix and one or more Drugs) is dissolved in a volatile organic solvent,

such as methylene chloride. The organic solution containing the drug is then suspended in an aqueous solution that contains a surface active agent such as poly(vinyl alcohol). The resulting emulsion is stirred until most of the organic solvent evaporated, leaving solid nanoparticles. The resulting nanoparticles are washed with water and dried overnight in a lyophilizer. Nanoparticles with different sizes and morphologies can be obtained by this method.

Drugs which contain labile polymers, such as certain polyanhydrides, may degrade during the fabrication process due to the presence of water. For these polymers, the following two methods, which are performed in completely anhydrous organic solvents, can be used.

2. Solvent Removal

Solvent removal can also be used to prepare particles from drugs that are hydrolytically unstable. In this method, the drug (or polymer matrix and one or more Drugs) is dispersed or dissolved in a volatile organic solvent such as methylene chloride. This mixture is then suspended by stirring in an organic oil (such as silicon oil) to form an emulsion. Solid particles form from the emulsion, which can subsequently be isolated from the supernatant. The external morphology of spheres produced with this technique is highly dependent on the identity of the drug.

3. Spray Drying

In this method, the drug (or polymer matrix and one or more Drugs) is dissolved in an organic solvent such as methylene chloride. The solution is pumped through a micronizing nozzle driven by a flow of compressed gas, and the resulting aerosol is suspended in a heated cyclone of air, allowing the solvent to evaporate from the microdroplets, forming particles. Particles ranging between 0.1-10 microns can be obtained using this method.

4. Phase Inversion

Particles can be formed from drugs using a phase inversion method. In this method, the drug (or polymer matrix and one or more Drugs) is dissolved in a "good" solvent, and the solution is poured into a strong non solvent for the drug to spontaneously produce, under favorable conditions, microparticles or nanoparticles. The method can be used to produce nanoparticles in a wide range of sizes, including, for example, about 100 nanometers to about 10 microns, typically possessing a narrow particle size distribution.

5. Coacervation

Techniques for particle formation using coacervation are known in the art, for example, in GB-B-929 406; GB-B-929 40 1; and U.S. Patent Nos. 3,266,987, 4,794,000, and 4,460,563. Coacervation involves the separation of a drug (or polymer matrix and one or more Drugs)solution into two immiscible liquid phases. One phase is a dense coacervate phase, which contains a high concentration of the drug, while the second phase contains a low concentration of the drug. Within the dense coacervate phase, the drug forms nanoscale or microscale droplets, which harden into particles. Coacervation may be induced by a temperature change, addition of a non-solvent or addition of a micro-salt (simple coacervation), or by the addition of another polymer thereby forming an interpolymer complex (complex coacervation).

6. Low Temperature Casting

Methods for very low temperature casting of controlled release microspheres are described in U.S. Patent No. 5,019,400 to Gombotz et al. In this method, the drug (or polymer matrix and sunitinib) is dissolved in a solvent. The mixture is then atomized into a vessel containing a liquid non-solvent at a temperature below the freezing point of the drug solution which freezes the drug droplets. As the droplets and non-solvent for the drug are warmed, the solvent in the droplets thaws and is extracted into the non-solvent, hardening the microspheres.

**D. Implants**

Implants can be formed which encapsulate and/or have dispersed therein the drug. In preferred embodiments, the implants are intraocular implants. Suitable implants include, but are not limited to, rods, discs, and wafers. The matrix can be formed of any of the non-biodegradable or biodegradable polymers described above, although biodegradable polymers are preferred. The composition of the polymer matrix is selected based on the time required for *in vivo* stability, *i.e.* that time required for distribution to the site where delivery is desired, and the time desired for delivery.
The implants may be of any geometry such as fibers, sheets, films, microspheres, spheres, circular discs, rods, or plaques. Implant size is determined by factors such as toleration for the implant, location of the implant, size limitations

in view of the proposed method of implant insertion, ease of handling, etc.

Where sheets or films are employed, the sheets or films will be in the range of at least about 0.5 mm x 0.5 mm, usually about 3 to 10 mm x 5 to 10 mm with a thickness of about 0.1 to 1.0 mm for ease of handling. Where fibers are employed, the fiber diameter will generally be in the range of about 0.05 to 3 mm and the fiber length will generally be in the range of about 0.5 to 10 mm.

The size and shape of the implant can also be used to control the rate of release, period of treatment, and drug concentration at the site of implantation. Larger implants will deliver a proportionately larger dose, but depending on the surface to mass ratio, may have a slower release rate. The particular size and geometry of the implant are chosen to suit the site of implantation.

Intraocular implants may be spherical or non-spherical in shape. For spherical-shaped implants, the implant may have a largest dimension (e.g., diameter) between about 5 μm and about 2 mm, or between about 10 μm and about 1 mm for administration with a needle, greater than 1 mm, or greater than 2 mm, such as 3 mm or up to 10 mm, for administration by surgical implantation. If the implant is non-spherical, the implant may have the largest dimension or smallest dimension be from about 5 μm and about 2 mm, or between about 10 μm and about 1 mm for administration with a needle, greater than 1 mm, or greater than 2 mm, such as 3 mm or up to 10 mm, for administration by surgical implantation.

The vitreous chamber in humans is able to accommodate relatively large implants of varying geometries, having lengths of, for example, 1 to 10 mm. The implant may be a cylindrical pellet (e.g., rod) with dimensions of about 2 mm x 0.75 mm diameter. The implant may be a cylindrical pellet with a length of about 7 mm to about 10 mm, and a diameter of about 0.75 mm to about 1.5 mm. In certain embodiments, the implant is in the form of an extruded filament with a diameter of about 0.5 mm, a length of about 6 mm, and a weight of approximately 1 mg. In some embodiments, the dimensions are, or are similar to, implants already approved for intraocular injection via needle: diameter of 460 microns and a length of 6 mm and diameter of 370 microns and length of 3.5 mm.

Intraocular implants may also be designed to be least somewhat flexible so as to facilitate both insertion of the implant in the eye, such as in the vitreous humor, and subsequent accommodation of the implant. The total weight of the implant is usually about 250 to 5000 μg, for example, about 500 - 1000 μg. In certain embodiments, the intraocular implant has a mass of about 500 μg, 750 μg, or 1000 μg.

2. Methods of Manufacture

Implants can be manufactured using any suitable technique known in the art. Examples of suitable techniques for the preparation of implants include solvent evaporation methods, phase separation methods, interfacial methods, molding methods, injection molding methods, extrusion methods, coextrusion methods, carver press method, die cutting methods, heat compression, and combinations thereof. Suitable methods for the manufacture of implants can be selected in view of many factors including the properties of the polymer/polymers present in the implant, the properties of the one or more drugs present in the implant, and the desired shape and size of the implant. Suitable methods for the preparation of implants are described, for example, in U.S. Patent No. 4,997,652 and U.S. Patent Application Publication No. US 2010/0124565.

In certain cases, extrusion methods may be used to avoid the need for solvents during implant manufacture. When using extrusion methods, the polymer/polymers and Drug are chosen so as to be stable at the temperatures required for manufacturing, usually at least about 85°C. However, depending on the nature of the polymeric components and the one or more Drugs, extrusion methods can employ temperatures of about 25°C to about 150°C, for example, about 65°C to about 130°C. Implants may be coextruded in order to provide a coating covering all or part of the surface of the implant. Such coatings may be erodible or non-erodible, and may be impermeable, semi-permeable, or permeable to the Drug, water, or combinations thereof. Such coatings can be used to further control release of the Drug from the implant.

Compression methods may be used to make the implants. Compression methods frequently yield implants with faster release rates than extrusion methods. Compression methods may employ pressures of about 50-150 psi, for example, about 70-80 psi, even more for example, about 76 psi, and use temperatures of about 0°C to about 115°C, for example, about 25°C.

## IV. Pharmaceutical Formulations

### A. Pharmaceutical Excipients

Pharmaceutical formulations contain sunitinib in combination with one or more pharmaceutically acceptable excipients. Representative excipients include solvents, diluents, pH modifying agents, preservatives, antioxidants, suspending agents, wetting agents, viscosity modifiers, tonicity agents, stabilizing agents, and combinations thereof. Suitable

pharmaceutically acceptable excipients are for example, selected from materials which are generally recognized as safe (GRAS), and may be administered to an individual without causing undesirable biological side effects or unwanted interactions.

Excipients can be added to the formulations to assist in sterility, preservations, and to adjust and/or maintain pH or isotonicity. Microparticles can be suspended in sterile saline, phosphate buffered saline ( PBS), Balanced salt solution( BSS), viscous gel or other pharmaceutically acceptable carriers for administration to the eye such as viscoelastic agents approved in the eye.

As noted above, drug release is affected by the media, especially by the pH of solutions. For example, release of sunitinib free base particles is faster in PBS at pH 7 than in saline solution since the free base forms salt, which is more hydrophilic than free base. Therefore the pH of the site of administration will have an effect on the drug release.

In some cases, the pharmaceutical formulation contains only one type of conjugate or polymeric particles for the controlled release of Drugs (e.g., a formulation containing drug particles wherein the drug particles incorporated into the pharmaceutical formulation have the same composition). In other embodiments, the pharmaceutical formulation contains two or more different type of conjugates or polymeric particles for the controlled release of Drugs (*e.g.,* the pharmaceutical formulation contains two or more populations of drug particles, wherein the populations of drug particles have different chemical compositions, different average particle sizes, and/or different particle size distributions).

Particles formed from the drugs will for example, be formulated as a solution or suspension for injection to the eye or into a tissue such as a tumor.

Pharmaceutical formulations for ocular administration are for example, in the form of a sterile aqueous solution or suspension of particles formed from sunitinib or pharmaceutically acceptable salt. Acceptable solvents include, for example, water, Ringer's solution, phosphate buffered saline (PBS), and isotonic sodium chloride solution. The formulation may also be a sterile solution, suspension, or emulsion in a nontoxic, parenterally acceptable diluent or solvent such as 1,3-butanediol.

In some instances, the formulation is distributed or packaged in a liquid form. Alternatively, formulations for ocular administration can be packed as a solid, obtained, for example by lyophilization of a suitable liquid formulation. The solid can be reconstituted with an appropriate carrier or diluent prior to administration.

Solutions, suspensions, or emulsions for ocular administration may be buffered with an effective amount of buffer necessary to maintain a pH suitable for ocular administration. Suitable buffers are well known by those skilled in the art and some examples of useful buffers are acetate, borate, carbonate, citrate, and phosphate buffers.

Solutions, suspensions, or emulsions for ocular administration may also contain one or more tonicity agents to adjust the isotonic range of the formulation. Suitable tonicity agents are well known in the art and some examples include glycerin, mannitol, sorbitol, sodium chloride, and other electrolytes.

Solutions, suspensions, or emulsions for ocular administration may also contain one or more preservatives to prevent bacterial contamination of the ophthalmic preparations. Suitable preservatives are known in the art, and include polyhexamethylenebiguanidine (PHMB), benzalkonium chloride (BAK), stabilized oxychloro complexes (otherwise known as Purite®), phenylmercuric acetate, chlorobutanol, sorbic acid, chlorhexidine, benzyl alcohol, parabens, thimerosal, and mixtures thereof.

Solutions, suspensions, or emulsions for ocular administration may also contain one or more excipients known art, such as dispersing agents, wetting agents, and suspending agents.

### B. Additional Active Agents

In addition to the sunitinib or pharmaceutically acceptable salt present in the polymeric particles, the formulation can contain one or more additional therapeutic, diagnostic, and/or prophylactic agents. The active agents can be a small molecule active agent or a biomolecule, such as an enzyme or protein, polypeptide, or nucleic acid. Suitable small molecule active agents include organic and organometallic compounds. In some instances, the small molecule active agent has a molecular weight of less than about 2000 g/mol, for example, less than about 1500 g/mol, for example, less than about 1200 g/mol. The small molecule active agent can be a hydrophilic, hydrophobic, or amphiphilic compound.

In some cases, one or more additional active agents may be encapsulated in, dispersed in, or otherwise associated with the particles. In certain embodiments, one or more additional active agents may also be dissolved or suspended in the pharmaceutically acceptable carrier.

In the case of pharmaceutical compositions for the treatment of ocular diseases, the formulation may contain one or more ophthalmic drugs. In particular embodiments, the ophthalmic drug is a drug used to treat, prevent or diagnose a disease or disorder of the posterior segment eye. Non-limiting examples of ophthalmic drugs include anti-glaucoma agents, anti-angiogenesis agents, anti-infective agents, anti-inflammatory agents, growth factors, immunosuppressant agents, anti-allergic agents, and combinations thereof.

Representative anti-glaucoma agents include prostaglandin analogs (such as travoprost, bimatoprost, and latanoprost),beta-andrenergic receptor antagonists (such as timolol, betaxolol, levobetaxolol, and carteolol), alpha-2

adrenergic receptor agonists (such as brimonidine and apraclonidine), carbonic anhydrase inhibitors (such as brinzolamide, acetazolamine, and dorzolamide), miotics (*i.e.,* parasympathomimetics, such as pilocarpine and ecothiopate), seretonergics muscarinics, dopaminergic agonists, and adrenergic agonists (such as apraclonidine and brimonidine).

Representative anti-angiogenesis agents include, but are not limited to, antibodies to vascular endothelial growth factor (VEGF) such as bevacizumab (AVASTIN®) and rhuFAb V2 (ranibizumab, LUCENTIS®), and other anti-VEGF compounds including aflibercept (EYLEA®); MACUGEN® (pegaptanim sodium, anti-VEGF aptamer or EYE001) (Eyetech Pharmaceuticals); pigment epithelium derived factor(s) (PEDF); COX-2 inhibitors such as celecoxib (CELEBREX®) and rofecoxib (VIOXX®); interferon alpha; interleukin-12 (IL-12); thalidomide (THALOMID®) and derivatives thereof such as lenalidomide (REVLIMID®); squalamine; endostatin; angiostatin; ribozyme inhibitors such as ANGIOZYME® (Sirna Therapeutics); multifunctional antiangiogenic agents such as NEOVASTAT® (AE-941) (Aeterna Laboratories, Quebec City, Canada); receptor tyrosine kinase (RTK) inhibitors such as sunitinib malate (SUTENT®); tyrosine kinase inhibitors such as sorafenib (Nexavar®) and erlotinib (Tarceva®); antibodies to the epidermal grown factor receptor such as panitumumab (VECTIBIX®) and cetuximab (ERBITUX®), as well as other anti-angiogenesis agents known in the art.

Anti-infective agents include antiviral agents, antibacterial agents, antiparasitic agents, and anti-fungal agents. Representative antiviral agents include ganciclovir and acyclovir. Representative antibiotic agents include aminoglycosides such as streptomycin, amikacin, gentamicin, and tobramycin, ansamycins such as geldanamycin and herbimycin, carbacephems, carbapenems, cephalosporins, glycopeptides such as vancomycin, teicoplanin, and telavancin, lincosamides, lipopeptides such as daptomycin, macrolides such as azithromycin, clarithromycin, dirithromycin, and erythromycin, monobactams, nitrofurans, penicillins, polypeptides such as bacitracin, colistin and polymyxin B, quinolones, sulfonamides, and tetracyclines.

In some cases, the active agent is an anti-allergic agent such as olopatadine and epinastine.

Anti-inflammatory agents include both non-steroidal and steroidal anti-inflammatory agents. Suitable steroidal active agents include glucocorticoids, progestins, mineralocorticoids, and corticosteroids.

The ophthalmic drug may be present in its neutral form, or in the form of a pharmaceutically acceptable salt. In some cases, it may be desirable to prepare a formulation containing a salt of an active agent due to one or more of the salt's advantageous physical properties, such as enhanced stability or a desirable solubility or dissolution profile. Generally, pharmaceutically acceptable salts can be prepared by reaction of the free acid or base forms of an active agent with a stoichiometric amount of the appropriate base or acid in water or in an organic solvent, or in a mixture of the two; generally, non-aqueous media like ether, ethyl acetate, ethanol, isopropanol, or acetonitrile are preferred. Pharmaceutically acceptable salts include salts of an active agent derived from inorganic acids, organic acids, alkali metal salts, and alkaline earth metal salts as well as salts formed by reaction of the drug with a suitable organic ligand (e.g., quaternary ammonium salts). Lists of suitable salts are found, for example, in Remington's Pharmaceutical Sciences, 20th ed., Lippincott Williams & Wilkins, Baltimore, MD, 2000, p. 704. Examples of ophthalmic drugs sometimes administered in the form of a pharmaceutically acceptable salt include timolol maleate, brimonidine tartrate, and sodium diclofenac. Non-limiting examples of pharmaceutically acceptable acids that can be used as the sunitinib counterion, include, but are not limited to, those derived from inorganic acids such as hydrochloric, hydrobromic, sulfuric, sulfamic, phosphoric, and nitric; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, mesylic, esylic, besylic, sulfanilic, 2-acetoxybenzoic, fumaric, toluenesulfonic, methanesulfonic, ethane disulfonic, oxalic, isethionic, and HOOC-(CH2)n-COOH where n is 0-4. In some cases, the active agent is a diagnostic agent imaging or otherwise assessing the eye. Exemplary diagnostic agents include paramagnetic molecules, fluorescent compounds, magnetic molecules, and radionuclides, x-ray imaging agents, and contrast media.

In certain embodiments, the pharmaceutical composition contains one or more local anesthetics. Representative local anesthetics include tetracaine, lidocaine, amethocaine, proparacaine, lignocaine, and bupivacaine. In some cases, one or more additional agents, such as a hyaluronidase enzyme, is also added to the formulation to accelerate and improves dispersal of the local anesthetic.

## V. Methods of Use

References to a method of treatment by therapy or surgery in the description are to be interpreted as references to compounds, pharmaceutical compositions and medicaments of the present invention for use in those methods.

Controlled release dosage formulations for the delivery of sunitinib or a pharmaceutically acceptable salt thereof can be used to treat a disease or disorder in a patient associated with vascularization, including cancer and obesity. In a preferred embodiment, the pharmaceutical compositions are administered to treat or prevent a disease or disorder in a patient associated with ocular neovascularization. Upon administration, the one or more drugs are released over an extended period of time at concentrations which are high enough to produce therapeutic benefit, but low enough to avoid cytotoxicity.

In order to treat chronic diseases of the eye, there is a need for long acting methods for delivering sunitinib or its pharmaceutically acceptable salt to the eye. Formulations which provide extended delivery of sunitinib or its salt will minimize the potential for toxicity associated with the administration of sunitinib. Formulations which provide extended delivery of sunitinib or its salt will also sustain suppression of VEGF and other stimulators of angiogenesis, maximize efficacy, promote regression of neovascularization, and minimize the potential for catastrophic complications including subretinal hemorrhage. In addition, reducing the need for frequent injections will decrease the risk of endophthalmitis and decrease the burden of frequent clinic visits, a major hardship for doctors, patients and their families.

**A. Diseases and Disorders of the eye**

When administered to the eye, the particles release a low dose of one or more active agents over an extended period of time, for example longer than 3, 7, 10, 15, 21, 25, 30, 45 days, or up to at least about 2 months, 3 months, 4 months, 5 months or 6 months or more The structure of the drug or makeup of the polymeric matrix, particle morphology, and dosage of particles administered can be tailored to administer a therapeutically effective amount of one or more active agents to the eye over an extended period of time while minimizing side effects, such as the reduction of scoptopic ERG b-wave amplitudes and/or retinal degeneration.

Pharmaceutical compositions containing particles for the controlled release of one or more Drugs can be administered to the eye of a patient in need thereof to treat or prevent one or more diseases or disorders of the eye. In some cases, the disease or disorder of the eye affects the posterior segment of the eye. The posterior segment of the eye, as used herein, refers to the back two-thirds of the eye, including the anterior hyaloid membrane and all of the optical structures behind it, such as the vitreous humor, retina, choroid, and optic nerve.

In preferred embodiments, a pharmaceutical composition containing particles administered to treat or prevent an intraocular neovascular disease. Eye diseases, particularly those characterized by ocular neovascularization, represent a significant public health concern. Intraocular neovascular diseases are characterized by unchecked vascular growth in one or more regions of the eye. Unchecked, the vascularization damages and/or obscures one or more structures in the eye, resulting in vision loss. Intraocular neovascular diseases include proliferative retinopathies, choroidal neovascularization (CNV), age-related macular degeneration (AMD), diabetic and other ischemia-related retinopathies, diabetic macular edema, pathological myopia, von Hippel-Lindau disease, histoplasmosis of the eye, central retinal vein occlusion (CRVO), comeal neovascularization, and retinal neovascularization (RNV). Intraocular neovascular diseases afflict millions worldwide, in many cases leading to severe vision loss and a decrease in quality of life and productivity.

Age related macular degeneration (AMD) is a leading cause of severe, irreversible vision loss among the elderly. Bressler, et al. JAMA, 291:1900-1901(2004). AMD is characterized by a broad spectrum of clinical and pathologic findings, such as pale yellow spots known as drusen, disruption of the retinal pigment epithelium (RPE), choroidal neovascularization (CNV), and disciform macular degeneration. AMD is classified as either dry (*i.e.,* non-exudative) or wet (*i.e.,* exudative). Dry AMD is characterized by the presence of lesions called drusen. Wet AMD is characterized by neovascularization in the center of the visual field. Although less common, wet AMD is responsible for 80%-90% of the severe visual loss associated with AMD (Ferris, et al. Arch. Ophthamol. 102:1640-2 (1984)). The cause of AMD is unknown. However, it is clear that the risk of developing AMD increases with advancing age. AMD has also been linked to risk factors including family history, cigarette smoking, oxidative stress, diabetes, alcohol intake, and sunlight exposure.

Wet AMD is typically characterized by CNV of the macular region. The choroidal capillaries proliferate and penetrate Bruch's membrane to reach the retinal pigment epithelium (RPE). In some cases, the capillaries may extend into the subretinal space. The increased permeability of the newly formed capillaries leads to accumulation of serous fluid or blood under the RPE and/or under or within the neurosensory retina. Decreases in vision occur when the fovea becomes swollen or detached. Fibrous metaplasia and organization may ensue, resulting in an elevated subretinal mass called a disciform scar that constitutes end-stage AMD and is associated with permanent vision loss (D'Amico D J. N. Engl. J. Med. 331:95-106 (1994)).

Other diseases and disorders of the eye, such as uveitis, are also difficult to treat using existing therapies. Uveitis is a general term referring to inflammation of any component of the uveal tract, such as the iris, ciliary body, or choroid. Inflammation of the overlying retina, called retinitis, or of the optic nerve, called optic neuritis, may occur with or without accompanying uveitis.

Ocular complications of uveitis may produce profound and irreversible loss of vision, especially when unrecognized or treated improperly. The most frequent complications of uveitis include retinal detachment, neovascularization of the retina, optic nerve, or iris, and cystoid macular edema. Macular edema (ME) can occur if the swelling, leaking, and background diabetic retinopathy (BDR) occur within the macula, the central 5% of the retina most critical to vision. ME is a common cause of severe visual impairment.

There have been many attempts to treat intraocular neurovascular diseases, as well as diseases associated with chronic inflammation of the eye, with pharmaceuticals. Attempts to develop clinically useful therapies have been plagued by difficulty in administering and maintaining a therapeutically effective amount of the pharmaceutical in the

ocular tissue for an extended period of time. In addition, many pharmaceuticals exhibit significant side effects and/or toxicity when administered to the ocular tissue.

Intraocular neovascular diseases are diseases or disorders of the eye that are characterized by ocular neovascularization. The neovascularization may occur in one or more regions of the eye, including the cornea, retina, choroid layer, or iris. In certain instances, the disease or disorder of the eye is characterized by the formation of new blood vessels in the choroid layer of the eye (*i.e.,* choroidal neovascularization, CNV). In some instances, the disease or disorder of the eye is characterized by the formation of blood vessels originating from the retinal veins and extending along the inner (vitreal) surface of the retina (*i.e.,* retinal neovascularization, RNV).

Exemplary neovascular diseases of the eye include age-related macular degeneration associated with choroidal neovascularization, proliferative diabetic retinopathy (diabetic retinopathy associated with retinal, preretinal, or iris neovascularization), proliferative vitreoretinopathy, retinopathy of prematurity, pathological myopia, von Hippel-Lindau disease, presumed ocular histoplasmosis syndrome (POHS), and conditions associated with ischemia such as branch retinal vein occlusion, central retinal vein occlusion, branch retinal artery occlusion, and central retinal artery occlusion.

The neovascularization can be caused by a tumor. The tumor may be either a benign or malignant tumor. Exemplary benign tumors include hamartomas and neurofibromas. Exemplary malignant tumors include choroidal melanoma, uveal melanoma or the iris, uveal melanoma of the ciliary body, retinoblastoma, or metastatic disease (e.g., choroidal metastasis).

The neovascularization may be associated with an ocular wound. For example, the wound may the result of a traumatic injury to the globe, such as a comeal laceration. Alternatively, the wound may be the result of ophthalmic surgery.

The drugs can be administered to prevent or reduce the risk of proliferative vitreoretinopathy following vitreoretinal surgery, prevent corneal haze following corneal surgery (such as corneal transplantation and eximer laser surgery), prevent closure of a trabeculectomy, or to prevent or substantially slow the recurrence of pterygii.

The drugs can be administered to treat or prevent an eye disease associated with inflammation. In such cases, the drug, for example, contains an anti-inflammatory agent. Exemplary inflammatory eye diseases include, but are not limited to, uveitis, endophthalmitis, and ophthalmic trauma or surgery.

The eye disease may also be an infectious eye disease, such as HIV retinopathy, toxocariasis, toxoplasmosis, and endophthalmitis.

Pharmaceutical compositions containing particles formed from one or more of the drugs can also be used to treat or prevent one or more diseases that affect other parts of the eye, such as dry eye, meibomitis, glaucoma, conjunctivitis (e.g., allergic conjunctivitis, vernal conjunctivitis, giant papillary conjunctivitis, atopic keratoconjunctivitis), neovascular glaucoma with iris neovascularization, and iritis.

1. Methods of Administration

The formulations described herein can be administered locally to the eye by intravitreal injection (e.g., front, mid or back vitreal injection), subconjunctival injection, intracameral injection, injection into the anterior chamber via the temporal limbus, intrastromal injection, injection into the subchoroidal space, intracorneal injection, subretinal injection, and intraocular injection. In a preferred embodiment, the pharmaceutical composition is administered by intravitreal injection.

The implants described herein can be administered to the eye using suitable methods for implantation known in the art. In certain embodiments, the implants are injected intravitreally using a needle, such as a 22-guage needle. Placement of the implant intravitreally may be varied in view of the implant size, implant shape, and the disease or disorder to be treated.

In some embodiments, the pharmaceutical compositions and/or implants described herein are co-administered with one or more additional active agents. "Co-administration", as used herein, refers to administration of the controlled release formulation of one or more Drugs with one or more additional active agents within the same dosage form, as well as administration using different dosage forms simultaneously or as essentially the same time. "Essentially at the same time" as used herein generally means within ten minutes, for example, within five minutes, for example, within two minutes, for example, within in one minute.

In some embodiments, the pharmaceutical compositions and/or implants described herein are co-administered with one or more additional treatments for a neovascular disease or disorder of the eye. In some embodiments, the pharmaceutical compositions and/or implants described herein are co-administered with one or more anti-angiogenesis agent such bevacizumab (AVASTIN®), ranibizumab, LUCENTIS®, or aflibercept (EYLEA®).

b. Dosage

Preferably, the particles will release an effective amount of sunitinib or a pharmaceutically acceptable salt thereof, over an extended period of time. In preferred embodiments, the particles release an effective amount of sunitinib

over a period of at least two weeks, over a period of at least four weeks, over a period of at least six weeks, over a period of at least eight weeks, over a period of three months, over a period of four months, over a period of five months or over a period of six months. In some embodiments, the particles release an effective amount of sunitinib over a period of three months or longer.

In some cases, a pharmaceutical formulation is administered to a patient in need thereof in a therapeutically effective amount to decrease choroidal neovascularization. In another embodiment, the pharmaceutical formulation is administered in an amount and for a time to decrease corneal neovascularization. In some cases, a pharmaceutical formulation is administered to a patient in need thereof in a therapeutically effective amount to decrease retinal neovascularization such as in acute macular degeneration (AMD)

c. Therapeutic Efficacy

The examples demonstrate methods for assessing therapeutic efficacy in various animal models. In the case of humans and animals such as dogs, the techniques are well established by those skilled in the ophthalmic field and would include slit lamp evaluation, visual inspection of the retina, measurement of field of vision, visual acuity, and intraocular pressure.

In the case of age-related macular degeneration, therapeutic efficacy in a patient can be measured by one or more of the following: assessing the mean change in the best corrected visual acuity (BCVA) from baseline to a desired time, assessing the proportion of patients who lose fewer than 15 letters (three lines) in visual acuity at a desired time as compared to a baseline, assessing the proportion of patients who gain greater than or equal to 15 letters (three lines) in visual acuity at a desired time as compared to a baseline, assessing the proportion of patients with a visual acuity Snellen equivalent of 20/2000 or worse at a desired time, assessing the National Eye Institute Visual Functioning Questionnaire, and assessing the size of CNV and the amount of leakage of CNV at a desired time using fluorescein angiography.

In certain embodiments, at least 25%, for example, at least 30%, for example, at least 35%, for example, at least 40% of the patients with recent onset CNV who are treated with the formulations described herein improve by three or more lines of vision.

The present invention will be further understood by reference to the following non-limiting examples.

## Example 1. Effects of surfactants, the form of sunitinib, and the overall alkalinity on the drug loading and *in vitro* release profiles of microparticles (MPs)

### Materials and Methods

*Materials - Two Forms of Sunitinib*

Two forms of sunitinib were used, i.e., sunitinib malmate and sunitinib free base, both acquired from LC Lab (Woburn, MA, USA).

Poly (D, L-lactic-co-glycolic acid (PLGA, 50:50), 2A was acquired from Alkermes, Waltham, MA, US; poly (D, L-lactic-co-glycolic acid (PLGA, 50:50), 2A from Lakeshore Biomaterials, Birmingham, Al, US; poly (D, L-lactic-co-glycolic acid (PLGA, 50:50), 4A from Lakeshore, Biomaterials, Birmingham, Al, US; poly (D, L-lactic-co-glycolic acid (PLGA, 75:25), PURASORB PDLG 7502A from PURAC, Netherlands; polyethylene glycol-Poly (D, L-lactic-co-glycolic acid), PEG-PLGA (5K, 45K), PEG 10%, PLGA 50:50, from Jinan Daigang Biomaterials Co, Ltd., Jinan, Shandong, China and purified by dissolved in chloroform and precipitated in ether. PLA, poly (D, L-lactic acid), polyvinyl alcohol (PVA) were acquired from Polyscience, Mw, 25000, hydrolysis 88%. PEG-PLA (5K, 45K), 10%PEG, PEG, 5K, was synthesized. N, N dimethy toludine, N, N dimethy aniline, malic acid citric acid, triethylamine, and all others from sigma (St. Louis, MO, USA)

*Preparation of Particles and Adjustine Acidity/Alkalinity*

Polymer microparticles loaded with two forms of sunitinib (either malate or free base) were prepared using a single o/w or s/o/w emulsion, solvent evaporation method. Briefly, a solution was made by mixing PLGA dissolved in methylene chloride (also known as dichloromethane, DCM) with drug dissolved in DMSO or drug suspend in methylence chloride(O/W). The mixture was homogenized (Silverson Homogenizer, model L4RT, Chesham Bucks, England) for 1 min into an aqueous solution containing 1% polyvinyl alcohol (PVA). The particles were then stirred for 2 hours to allow hardening, collected by centrifugation, washed with double distilled water and freeze-dried.

In selecting surfactants, both cationic and ionic surfactants, sodium dodecyl sulfate ("SDS") and hexadecyltrimethylammonium bromide ("HDTA"), were first attempted and added to the solvents to make the particles. Alternatively,

a non-ionic solvent, polyvinyl alcohol (PVA) was used in substitution of SDS or HDTA.

Particles can also be made by w/o/w or s/w/o/w double motion method. Briefly, a solution was made by mixing drug in DMSO and water, or drug suspended in water, then add to PLGA in methylene chloride, sonicate, then the mixture was homogenized (Silverson Homogenizer, model L4RT, Chesham Bucks, England) for 1 min into an aqueous solution containing 1% polyvinyl alcohol (PVA). The particles were then stirred for 2 hours to allow hardening, collected by centrifugation, washed with double distilled water and freeze-dried.

The acidity of the organic phase can be adjusted to increase the drug loading capability of formed microparticles by adding alkaline to organic phase.

*Formulation IDs*

For ease of identification, particles prepared according to different formulations were each given an ID, e.g. *MP-n* (n is a number from 1 to 20). Sunitinib malate was used in *MP-1 to MP-10, MP-14, MP-15 and MP-18*; whereas sunitinib free base was used in *MP-11 to MP-13, MP-16, MP-17, MP-19,* and *MP-20.*

*Oil in water (O/W) single emulsion method*

*MP-1*: 100mg PEG-PLGA (5K, 45K) was dissolved in 1 mL methylene chloride, and 20mg sunitinib malate was dissolved in 0.5 mL DMSO and triethylamine. They were then mixed together, homogenized at 5000rpm, 1 min. into an aqueous solution containing 1% polyvinyl alcohol (PVA) and stirred for 2 hours, particles collected, washed with double distilled water, and freeze dried.

*MP-2*: 200mg PLGA (2A, Alkermers), was dissolved in 3 mL methylene chloride, and 40mg sunitinib malate was dissolved in 0.5 mL DMSO and triethylamine. They were then mixed together and homogenized at 5000rpm, 1 min in 1% PVA and stir for 2 hours, particles collected, washed with double distilled water, and freeze dried.

*MP-3*: 180mg PLGA (2A, Alkermers), and 20mg PEG-PLGA( 5K, 45K) was dissolved in 3 mL methylene chloride, and 40mg sunitinib malate dissolved in 0.5 mL DMSO, triethyamine. They were then mixed together, homogenized at 5000rpm, 1 min in 1% PVA and stirred for 2 hours, particles collected, washed with double distilled water, and freeze dried.

*MP-4*: 140mg PLGA (2A, Alkermers), 60mg PEG-PLGA( 5K, 45K) was dissolved in 3 mL methylene chloride, and 40mg sunitinib malate dissolved in 0.5 mL DMSO and triethylamine. They were then mixed together, homogenized at 5000rpm, 1 min in 1% PVA and stirred for 2 hours, particles collected, washed with double distilled water, and freeze dried.

*MP-5*: 100mg PLGA (2A, Alkermers) and 100 mg PEG-PLGA( 5K, 45K) were dissolved in 3 mL methylene chloride, and 40mg sunitinib malate dissolved in 0.5 mL DMSO and triethylamine. They were then mixed together, homogenized at 5000rpm, 1 min in 1% PVA and stirred for 2 hours, particles collected, washed with double distilled water, and freeze dried.

*MP-6*: 90 mg PLGA (2A, Alkermers), and 10 mg PEG-PLGA( 5K, 45K) were dissolved in 1 mL methylene chloride, and 20mg sunitinib malate dissolved in 0.25 mL DMSO, and N, N-dimethyl toludine. They were then mixed together, homogenized at 5000rpm, 1 min in 1% PVA and stirred for 2 hours, particles collected, washed with double distilled water, and freeze dried.

*MP-7*: 90 mg PLGA (2A, Alkermers) and 10 mg PEG-PLGA( 5K, 45K) were dissolved in 1 mL methylene chloride, and 20mg sunitinib malate dissolved in 0.25 mL DMSO and N, N-dimethy aniline. They were then mixed together, homogenized at 5000rpm, 1 min in 1% PVA and stirred for 2 hours, particles collected, washed with double distilled water, and freeze dried.

*MP-8*: 160 mg PLGA (2A, Alkermers) and 40 mg PEG-PLGA( 5K, 45K) were dissolved in 2 mL DMF, 20mg sunitinib malate was added and votexed, then homogenized at 5000rpm, 1 min in 1% PVA and stirred for 2 hours, particles collected, washed with double distilled water, and freeze dried.

*MP-9*: 90 mg PLGA (4A, Lakeshore biomaterials) and 10 mg PEG-PLGA( 5K, 45K) were dissolved in 1 mL methylene chloride, 20mg sunitinib malate dissolved in in 0.25 mL DMSO, 0.1M potassium hydroxyl in ethanol added, homogenized at 5200rpm, 1 min in 1% PVA and stirred for 2 hours, collect particles, washed with double distilled water, and freeze dried.

*MP-10*: 160mg PLGA (2A, Alkermers) and 40 mg PEG-PLGA( 5K, 45K) were dissolved in 2 mL methylene chloride, 40mg sunitinib malate dissolved in in 1 mL DMSO, triethylamine added, homogenized at 5000rpm, 1 min in 1% PVA and stirred for 2 hours, particles collected, washed with double distilled water, and freeze dried.

*MP-11*: 100mg PLGA (4A, Lakeshore biomaterials), dissolved in 1 mL methylene chloride, 20mg sunitinib free base dissolved in 0.25mL DMSO, a small amount of 1% acetic acid in ethanol added, homogenized at 5000rpm, 1 min

in 1% PVA and stirred for 2 hours, particles added, washd with double distilled water, and freeze dried.
*MP-12*: 100mg PLGA (7502A, PURAC, Nethanlands) was dissolved in 2 mL methylene chloride, and 20mg sunitinib free base dissolved in 0.25mL DMSO, 0.1M citric acid in ethanol added, homogenized at 3000rpm, 1 min in 1% PVA and stirred for 2 hours, particles collected, washed with double distilled water, and freeze dried.
*MP-13*: 100mg PLGA (4A, Lakeshore biomaterials) was dissolved in 2 mL methylene chloride, and 20mg sunitinib free base dissolved in 0.25 mL DMSO, 0.1M malic acid in ethanol, homogenized at 3000rpm, 1 min in 1% PVA and stirred for 2 hours, particles collected, washed with double distilled water, and freeze dried.

*S/O/W (solid in oil in water) single emulsion method*

*MP-14*: 90 mg PLGA (4A, Lakeshore biomaterials) and 10 mg PEG-PLGA( 5K, 45K)) were dissolved in 2 mL methylene chloride, 20mg sunitinib malate added and sonicated, then poured in 1% PVA to homogenize 1 min at 4300 rpm, stirred for 2 hours, particles collected, washed with double distilled water, and freeze dried.
*MP-15* (Controlled water phase pH): 90 mg PLGA (4A, Lakeshore biomaterials), 10 mg PEG-PLGA( 5K, 45K)), were dissolved in 1 mL methylene chloride, 20mg sunitinib malate added to above solution, sonicated, then poured into 1% PVA PBS (phosphate buffer solution pH=7.4), homogenized 1 min at 4800rpm and stirred for 2 hours, particles collected, washed with double distilled water, and freeze dried.
*MP-16:* 90 mg PLGA (4A, Lakeshore biomaterials) and 10 mg PEG-PLGA( 5K, 45K) were dissolved in 1 mL methylene chloride, 20mg sunitinib free base added to above solution, 0.1 M malic acid in ethanol added to above solution, the solution sonicated, then poured into 1% PVA to homogenize 1 min at 4800rpm and stirred for 2 hours, particles collected, washed with double distilled water, and freeze dried.
*MP-17*: 90 mg PLGA (4A, Lakeshore biomaterials) and 10 mg PEG-PLGA( 5K, 45K)) were dissolved in 1 mL methylene chloride, 20mg sunitinib free base added to above solution, then poured into 1% PVA in PBS and homogenized 1 min at 4800 rpm and stirred for 2 hours, particles collected, washed with double distilled water, and freeze dried.

*Double emulsion (w/o/w) method*

*MP-18*: 90 mg PLGA (4A, Lakeshore biomaterials) and 10 mg PEG-PLGA( 5K, 45K)) was dissolved in 1 mL methylene chloride, 20 mg sunitinib malate was added to 100 μl DMSO and 200 μl water, sonicated, then poured into 1% PVA in PBS and homogenized 1 min at 3000 rpm and stirred for 2 hours, particles collected, washed with double distilled water, and freeze dried.
MP-19: 90 mg PLGA (4A, Lakeshore biomaterials) and 10 mg PEG-PLGA( 5K, 45K)) were dissolved in 1 mL methylene chloride, 20mg sunitinib free base was added in 100 μl DMSO and 200 μl water, sonicated, then poured into 1% PVA in PBS and homogenized 1 min at 4000 rpm and stirred for 2 hours, particles collected, washed with double distilled water, and freeze dried.
MP-20: 90 mg PLGA (4A, Lakeshore biomaterials) and 10 mg PEG-PLGA( 5K, 45K)) were dissolved in 1 mL methylene chloride, 20mg sunitinib free base was added in 100 μl DMSO and 200 μl water, 0.1 M malic acid added, sonicated, then poured into 1% PVA and homogenized 1 min at 4000 rpm and stirred for 2 hours, particles collected, washed with double distilled water, and freeze dried.

Characterization of Microparticles (MPs)

The size of MPs was determined using a Coulter Multisizer VI (Beckman-Coulter Inc., Fullerton, CA). Approximately 2 mL of isoton II solution was added to 5-10 mg microparticles. The solution was briefly vortexed to suspend the microparticles and then added drop-wise to 100 mL of isoton II solution until the coincidence of particles was between 8% and 10%. Greater than 100,000 particles were sized for each batch of microparticles to determine the mean particle size. To determine the drug release rate in vitro, 5 mg of drug-loaded particles were suspended in 1 mL of phosphate-buffered saline (pH 7.4) and incubated at 37°C on a rotator. At selected time points, microparticles were precipitated by centrifugation, the supernatant removed and replaced with fresh phosphate buffer.
The drug loading was determined by UV-Vis spectrophotometry. Microparticles containing sunitinib (10 mg total weight) were dissolved in anhydrous DMSO (1 mL) and further diluted until the concentration of drug was in the linear range of the standard curve of UV absorbance of the drug. The concentration of the drug was determined by comparing the UV absorbance to a standard curve. Drug loading is defined as the weight ratio of drug to microparticles.

The *in vitro* drug release was determined by suspending MPs containing sunitinib (10 mg total weight) in 4 mL of PBS containing 1% Tween 20 in a 6-mL glass vial and incubated at 37 oC under shaking at 150 rpm. At predetermined time points, 3 mL of the supernatant was withdrawn after particles settled to the bottom of the vial and replaced with 3 mL of fresh release medium. The drug content in the supernatant was determined by UV-Vis spectrophotometry or HPLC.

## Results

### Summary of Release Results

All sunitinib (F127) was linearly released over a period of approximately 30 days in PBS (pH 7.4) of PLGA sunitinib particles made by F127.

All sunitinib (F127/PVA) was linearly released in a period of approximately 40 days in PBS (pH 7.4) from PLGA particles made by PVA, and then washed by F-127

All sunitinib was linearly released over a period of approximately 60 to 70 days in PBS (pH 7.4) from PLGA particles made by PVA.

All sunitinib was linearly released over approximately 100-120 days in PBS (pH 7.4) from PEG-PLGA particles.

All sunitinib was linearly released over a period of approximately 120 days in PBS (7.4) from PEG-PLA particles.

### Summary of Effect of Surfactants and pH on Loading

Both cationic and ionic surfactants yielded extremely low loadings, e.g., 0.20% with SDS and 0.27 % with HDTA bromide. Substituting PVA increased loading up to 1.1%. Sunitinib free base crystallized and could not be utilized to obtain higher loading. Adding DMSO to the solvent increased loading even more so, up to around 5%. However, an increased loading up to 16.1% loading capability with PEG-PLGA was achieved by increasing the alkalinity of the sunitinib solution, which could be further increased by adding DMF, compared to the loading capability of only 1% with no alkaline added.

Table 1 shows the sizes, drug loading capabilities, and first day release percentages of MP formulations prepared using PVA, two forms of subnitinib, and different alkalinity of the overall solution as described above.

**Table 1: Microparticles Formulation Summary**

| Lot ID | Polymer | First day Release (%) | Yield (%) | Drug loading (%) | Size before freezing drying | Size after freezing drying |
|---|---|---|---|---|---|---|
| MP-1 | PEG-PLGA(SK,45K, Shangdong) 10%(PEG) | 25.6 | 49 | 16.1 | 31.28±11.4 | 31.40±11.1 |
| MP-2 | PLGA(Alkermer 2A) | 2.7 | 51 | 10.3 | 12.34±5.16 | 10.40±4.71 |
| MP-3 | PEG-PLGA(5K,45K Shangdong) 10% /PLGA(90%) (Alkermer 2A) 1% (PEG) | 2.8 | 58 | 10.1 | 17.33±10.7 | 13.03±4.74 |
| MP-4 | PEG-PLGA(5K,45K, Shangdong) 30%) /PLGA (70%)(Alkermer 2A) 3%(PEG) | 6.8 | 61 | 11.3 | 24.64±10.2 | 26.9±9.12 |
| MP-5 | PEG-PLGA (5K,45K, Shangdong) (50%) /PLGA(50%)(Alkermer 2A) 5%(PEG) | 8.7 | 56 | 12.6 | 35.49±13.1 | 31.75±10.4 |
| MP-6 | PEG-PLGA(5K,45K Shangdong) 10% /PLGA(90%) (Alkermer 2A) 1% (PEG) | 6.1 | 48 | 11.8 | 22.90±7.2 | 24.23±8.54 |
| MP-7 | PEG-PLGA(5K,45K, ) Shangdong 10% /PLGA(90%) (Alkermer 2A) 1% (PEG) | 5.8 | 51 | 9.9 | 13.68±6.59 | 13.53±6.53 |

(continued)

| Lot ID | Polymer | First day Release (%) | Yield (%) | Drug loading (%) | Size before freezing drying | Size after freezing drying |
|---|---|---|---|---|---|---|
| MP-8 | PEG-PLGA(5K,45K Shangdong,) 20% /PLGA(80%) (Alkermer 2A) 2% (PEG) | 18.7 | 35 | 10.0 | 26.72±7.66 | 26.96±8.38 |
| MP-9 | PEG-PLGA(5K,45K, Shangdong) 10% /PLGA(90%)(Lakeshare 4A) 1%(PEG) | 51.3 | 31 | 13.9 | 31.53±9.80 | 33.81±8.90 |
| MP-10 | PEG-PLGA(5K,45K, Shangdong) 20% /PLGA(80%) (Alkermer 2A) 2% (PEG) | 28.4 | 43 | 15.1 | 17.33±10.7 | 18.51±10.7 |
| MP-11 | PLGA (Lakeshare 4A) | 16.1 | 43 | 13.7 | 12.78±6.84 | 12.29±7.03 |
| MP-12 | PLGA(PURAC, 7502A) | 8.3 | 52 | 13.9 | 11.91±5.77 | 11.51±5.0 |
| MP-13 | PLG (Lakeshare 4A) | 9.4 | 42 | 11.6 | 16.02±9.94 | 16.0±10.2 |
| MP-14 | PEG-PLGA(SK,45K, Shangdong) 10% /PLGA(90%)(Lakeshare 4A) 1%(PEG) | 6.1 | 41 | 11.2 | 16.92±5.36 | 16.96±5.61 |
| MP-15 | PEG-PLGA(5K,45K, Shangdong) 10% /PLGA(90%)(Lakeshare 4A) 1%(PEG) | 10.2 | 64 | 15.1 | 18.31±7.78 | 18.54±8.33 |
| MP-16 | PEG-PLGA(5K,45K, Shangdong) 10% /PLGA(90%)(Lakeshare 4A) 1%(PEG) | 1.9 | 43 | 15.9 | 19.81±8.71 | 22.25±10.7 |
| MP-17 | PEG-PLGA(5K,45K, ) Shangdong 10% /PLGA(90%)(Lakeshare 4A) 1%(PEG) | 1.2 | 53 | 14.3 | 18.28±7.36 | 18.65±7.63 |
| MP-18 | PEG-PLGA(SK,45K, Shangdong) 10% /PLGA(90%)(Lakeshare 4A) 1%(PEG) | 19.6 | 57 | 12.8 | 35.69±11.6 | 34.88±12.0 |
| MP-19 | PEG-PLGA(5K,45K, Shangdong) 10% /PLGA(90%)(Lakeshare 4A) 1%(PEG) | 7.08 | 40 | 14.5 | 41.95±10.7 | 39.88±10.1 |
| MP-20 | PEG-PLGA(5K,45K Shangdong,) 10% /PLGA(90%)(Lakeshare 4A) 1%(PEG) | 11.8 | 51 | 12.5 | 34.40±10.8 | 33.55±13.0 |

**Example 2: Preparation and *in vitro* release profiles of nanoparticles (NPs) encapsulating sunitinib**

**Materials and Methods**

*Formulation IDs*

For ease of identification, nanoparticles prepared in an aqueous phase containing 1% of PVA in PBS (pH 7.4) according to different formulations were each given an ID, e.g. *NP-n* (n is a number from 21 to 27).

*NP-21:* 100 mg PLGA (2A, Lakeshore biomaterials) was dissolved in 1 mL methylene chloride. 20mg sunitinib

malmate was added in 250 μl DMSO and then poured into 1% PVA. 5mL was sonicated 3 mins and poured into 80mL 0.1 % PVA, stirred for 2 hours, particles collected, washed with double distilled water, and freeze dried.

*NP-22:* 100 mg PLGA (2A, Lakeshore biomaterials) was dissolved in 1 mL methylene chloride. 20 mg sunitinib malmate was added in 250 μl DMSO and then poured into 1% PVA in PBS( 7.4). 5mL was sonicated for 3 mins and poured into 80mL 0.1 % PVA, stirred for 2 hours, particles collected, washed with double distilled water, and freeze dried.

*NP-23:* 100 mg PLGA (1A, Lakeshore biomaterials) was dissolved in 1 mL methylene chloride. 20 mg sunitinib malmate was added in 250 μl DMSO and then poured into 1% PVA in PBS ( 7.4). 5mL was sonicated 3mins and poured into 80mL 0.1 % PVA in PBS, stirred for 2 hours, particles collected, and washed with double distilled water, freeze dried.

*NP-24:* 100 mg PLGA (75:25, 4A, Lakeshore biomaterials) was dissolved in 1 mL methylene chloride. 20 mg sunitinib malmate was added in 250 ul DMSO and then poured into 1% PVA in PBS (7.4). 5mL was sonicated for 3 mins and poured into 80mL 0.1 % PVA in PBS, stirred for 2 hours, particles collected, washed with double distilled water, and freeze dried.

*NP-25:* 100 mg PLGA (2A, Resomer biomaterials) was dissolved in 1 mL methylene chloride. 20 mg sunitinib malmate was added in 250 μl DMSO, TEA 20 μl added and then poured into 1% PVA. 5 mL was sonicated 3 mins and poured into 80mL 0.1 % PVA, stirred for 2 hours, particles collected, washed with double distilled water, and freeze dried.

*NP-26:* 100 mg PLGA (2A, Resomer biomaterials) was dissolved in 1 mL methylene chloride. 20mg sunitinib malmate was added in 250 μl DMSO, and then poured into 1% PVA in PBS (7.4). 5mL was sonicated 3mins and poured into 80mL 0.1 % PVA in PBS ( 7.4), stirred for 2 hours, particles collected, washed with double distilled water, and freeze dried.

*NP-27:* 100 mg PEG-PLGA(5K,45K Shangdong, 10% PEG) was dissolved in 1 mL methylene chloride. 20mg sunitinib malmate was added in 250 μl DMSO, and then poured into 1% PVA, sonicated 3mins and poured into 80mL 0.1 % PVA, stirred for 2 hours, particles collected, washed with double distilled water, and freeze dried.

## Results

**Table 2: Nanoparticles Formulation Summary**

| Lot ID | Polymer | First day Release (%) | Yield (%) | Drug loading (%) | Size before freezing drying( nm) | Size after freezing drying (nm) |
|---|---|---|---|---|---|---|
| NP-21 | PLGA (Lakeshare 2A) | 12.1 | 39 | 8.1 | 133.8±9.6 | 133.2±5.5 |
| NP-22 | PLGA (Lakeshare 2A) | 15.3 | 48 | 9.2 | 187.9±12.8 | 194.7±13.1 |
| NP-23 | PLGA (Lakeshare 1A) | 11.6 | 42 | 12.1 | 185.7±5.5 | 195.3±6.8 |
| NP-24 | PLGA (75:25) (Lakeshare 4A) | 21.5 | 44 | 9.1 | 174.2±8.6 | 181.5±25.7 |
| NP-25 TEA | PLGA ( Rosemer 2A) | 15.1 | 39 | 8.1 | 151.8±12.0 | 169.2±18.1 |
| NP-26 | PLGA ( Rosemer 2A) | 8.3 | 46 | 7.6 | 126.9±10.1 | 136±12.5 |
| NP-27 | PEG-PLGA (5K,45K) Shangdong 10%PEG | 15.6 | 41 | 3.7 | 206.5±14.6 | 212.5±6.7 |

**Example 3. Effect of aqueous pH on encapsulation efficiency of sunitinib**

**Materials and Methods**

Polymer microparticles of PLGA and/or a diblock copolymer of PLGA and PEG covalently conjugated to PLGA ($M_w$ 45 kDa) (PLGA45k-PEG5k) with or without sunitinib malate were prepared using a single emulsion solvent evaporation method. Briefly, PLGA and/or PLGA-PEG were first dissolved in dichloromethane (DCM) and sunitinib malate was dissolved in dimethyl sulfoxide (DMSO) at predetermined concentrations. The polymer solution and the drug solution were mixed to form a homogeneous solution (organic phase). The organic phase was added to an aqueous solution of 1% polyvinyl alcohol (PVA) (Polysciences, Mw 25 kDa, 88% hydroplyzed) and homogenized at 5,000 rpm for 1 min using an L5M-A laboratory mixer (Silverson Machines Inc., East Longmeadow, MA) to obtain an emulsion.

The solvent-laden microparticles in the emulsion were then hardened by stirring at room temperature for >2 hr to allow the DCM to evaporate. The microparticles were collected by sedimentation and centrifugation, washed three times in water and dried by lyophilization.

As the solubility of sunitinib in aqueous solution was shown to be pH dependent, microparticle (MP) formulations encapsulating sunitinib were prepared in aqueous phases of various pH values (as shown in Table 3) to investigate the effect of aqueous pH on drug encapsulation.

**Table 3: Preparation of MPs at different aqueous pHs.**

| Formulation ID | Organic phase | | | | | | Aqueous phase | | Emulsion rate (rpm) |
|---|---|---|---|---|---|---|---|---|---|
| | PLGA (mg) | PLGA type | PLGA 5050-PEG 5kD (10% PEG by wt) (mg) | DCM (mL) | Sunitinib malate (mg) | DMSO (mL) | Surfactant | Volume (mL) | |
| DC-2-55-2 | 560 | 7525 4A | 5.6 | 4 | 90 | 2 | 1% PVA in pH 4 buffer | 200 | 5000 |
| DC-2-55-3 | 560 | 7525 4A | 5.6 | 4 | 90 | 2 | 1% Borate Buffer (pH 10) | 200 | 5000 |
| DC-2-55-4 | 560 | 7525 4A | 5.6 | 4 | 90 | 2 | 1% PVA in H2O (pH 6) | 200 | 5000 |
| DC-2-55-5 | 560 | 7525 4A | 5.6 | 4 | 90 | 2 | 1% PVA in PBS (pH 7.4) | 200 | 5000 |

Determination of drug loading

Drug loading was determined by UV-Vis spectrophotometry. Microparticles containing sunitinib (10 mg total weight) were dissolved in anhydrous DMSO (1 mL) and further diluted until the concentration of drug was in the linear range of the standard curve of UV absorbance of the drug. The concentration of the drug was determined by comparing the UV absorbance to a standard curve. Drug loading is defined as the weight ratio of drug to microparticles.

Measurement of average size and size distribution of microparticles

Several milligrams of the microparticles were first suspended in water and dispersed in an ISOTON® diluent. The mean particle size and distributions were determined using a COULTER MULTISIZER IV (Beckman Coulter, Inc., Brea, CA).

**Results**

Table 4 shows that the loading and encapsulation efficiency of MPs prepared according to the formulations in

Table 3. The efficiency of drug loading and encapsulation increased significantly when the aqueous pH was increased from 4 to 7.4, and more substantially from 6 to 7.4. However, when the pH was adjusted to 10 and the aqueous solution became more basic, the morphology of many particles changed from spherical to irregular shapes and some particles formed aggregates, suggesting aqueous solution of high pH is also unfavorable for producing particles of high loading of sunitinib and high quality. Hence, the preferred range of aqueous pH is between 6 and 10, and more preferably between 6 and 8.

**Table 4: The effect of aqueous phase pH on encapsulation efficiency of sunitinib**

| Sample ID | Aqueous pH | Actual loading | Target loading | Encapsulation efficiency | Mean diameter (μm) |
|---|---|---|---|---|---|
| DC-2-55-2 | 4 | 3.1% | 13.7% | 22% | 27.0 ± 7.9 |
| DC-2-55-4 | 6 | 5.0% | 13.7% | 36% | 28.0 ± 8.3 |
| DC-2-55-5 | 7.4 | 11.5% | 13.7% | 84% | 27.4 ± 7.6 |
| DC-2-55-3 | 10 | NA | 13.7% | NA | NA |

**Example 4. Effects of polymer concentration and polymer viscosity on encapsulation efficiency of sunitinib at aqueous pH 7.4**

**Materials and Methods**

Microparticle (MP) formulations encapsulating sunitinib were prepared in aqueous phosphate buffered saline (PBS, pH 7.4), as shown in Table 5.

**Table 5: Preparation of MPs at different polymer concentrations or using polymers with different molecular weights.**

| ID | Organic phase | | | | | | Aqueous phase | | Emulsion rate (rpm) |
|---|---|---|---|---|---|---|---|---|---|
| | PLGA (mg) | PLGA type | Formulation PLGA 5050-PEG 5kD (10% PEGby wt) (mg) | DCM (mL) | Sunitinib malate (mg) | DMSO (mL) | Surfactant | Volume (mL) | |
| | | | | | | | | | |
| DC-2-50-1 | 800 | 7525 4A | 8 | 4 | 145 | 2 | 1% PVA in PBS | 200 | 5000 |
| DC-2-50-2 | 560 | 7525 4A | 5.6 | 4 | 100 | 2 | 1% PVA in PBS | 200 | 5000 |
| DC-2-50-3 | 400 | 7525 4A | 4 | 4 | 70 | 2 | 1% PVA in PBS | 200 | 5000 |
| DC-2-50-4 | 280 | 7525 4A | 2.8 | 4 | 50 | 2 | 1% PVA in PBS | 200 | 5000 |
| DC-2-50-5 | 200 | 7525 4A | 2 | 4 | 35 | 2 | 1% PVA in PBS | 200 | 5000 |
| DC-1-53-1 | 400 | 7525 6E | 4 | 4 | 160 | 2 | 1% PVA in PBS | 200 | 5000 |
| DC-1-53-2 | 400 | 8515 6E | 4 | 4 | 160 | 2 | 1% PVA in PBS | 200 | 5000 |
| DC-1-53-3 | 400 | 8515 6A | 4 | 4 | 160 | 2 | 1% PVA in PBS | 200 | 5000 |

**Results**

Table 6 shows that the loading and encapsulation efficiency of MPs prepared according to the formulations in

Table 5. The drug loading and encapsulation efficiency increased as the concentration of polymer PLGA 7525 4A increased from 50.5 mg/mL to 202 mg/mL (Figure 1, Figure 2B, and Table 5). This is likely due to the fact that at higher concentrations, the polymer solution is more viscous and functions effectively as a barrier preventing drug molecules from diffusing into the aqueous phase. Particularly, the encapsulation efficiency increased to over 50% at 100 mg/mL polymer concentration. The dynamic viscosity of this polymer solution in DCM (methylene chloride), prior to mixing with sunitninb malate solution in DMSO, was estimated to be around 350 cPs. It is believed the preferred minimal viscosity of polymer solution in DCM is about 350 cPs, and preferably the polymer viscosity is around 720 cPs by calculation (which correlates to polymer concentration of 140 mg/mL in DCM). The mean diameter of the microparticles also increased as a function of the polymer concentration. At a polymer concentration of 200 mg/mL and an aqueous pH of 7.4, the encapsulation efficiency was as high as 92%.

The drug loading and encapsulation efficiency also increased as the viscosity of polymer solutions increased at a given polymer concentration and at a given aqueous pH of 7.4. At the same concentration of 100 mg/mL, the viscosity of PLGA 75:25 6E solution, of PLGA 85:15 6E solution, and of PLGA 85:15 6A solution is higher than that of PLGA 75:25 4A solution, because polymer PLGA 75:25 6E, polymer PLGA 85:15 6E, and polymer PLGA 85:15 6E each has a higher molecular weight than polymer PLGA 75:25 4A. Specifically, the viscosity of 100 mg/mL PLGA 75:25 6E in DCM was calculated to be about 830 cPs. As a result, the encapsulation efficiency of the PLGA 75:25 6E, PLGA 85:15 6E, or PLGA 85:15 6A formulation was about 80%, which was higher than that of the formulation containing the same concentration of PLGA 7525 4A at 53% (Table 6).

**Table 6: Relationship between polymer concentration/viscosity and encapsulation efficiency for sunitinib**

| Sample ID | PLGA | PLGA-PEG | Polymer conc. (mg/mL) | Aqueous pH | Actual loading | Target loading | Encapsulation efficiency |
|---|---|---|---|---|---|---|---|
| DC-2-50-5 | 99% PLGA 7525 4A | 1% PLGA-PEG 5kD (10% PEG) | 50.5 | 7.4 | 4.9% | 15% | 33% |
| DC-2-50-4 | | | 70.7 | | 6.8% | 15% | 45% |
| DC-2-50-3 | | | 101 | | 7.8% | 15% | 53% |
| DC-2-50-2 | | | 141.4 | | 12.0% | 15% | 80% |
| DC-2-50-1 | | | 202 | | 13.9% | 15% | 91% |
| DC-1-53-1 | 99% PLGA 7525 6E | | 101 | | 23.7% | 28% | 84% |
| DC-1-53-2 | 99% PLGA 8515 6E | | 101 | | 23.9% | 28% | 84% |
| DC-1-53-3 | 99% PLGA 8515 6A | | 101 | | 22.6% | 28% | 80% |

The results indicate that the drug loading and encapsulation efficiency of sunitinib formulations can be significantly improved by modifying the polymer concentration/viscosity at an optimized aqueous pH. It is believed possible to use polymer solution that is even more viscous than the ones described above. However, at certain point the solution would be too viscous to mix thoroughly with the aqueous phase and to form microparticles with relatively uniform size distribution.

**Example 5. Durations of release of sunitinib-enapsulated polymer** microparticle **formulations formed at aqueous pH 7.4.**

**Materials and Methods**

*Formulations*

The following microparticle (MP) formulations were prepared: formulation ID: DC-1-53-1, DC-1-53-2, and DC-1-53-3 according to Table 5; and formulations according to Table 7. The drug loading and encapsulation efficiency of the formulations in Table 7 were assayed as previously described in Example 3.

**Table 7: Preparation of more MPs for *in vitro* release assays.**

| Formulation ID | Organic phase | | | | | | Aqueous phase | | Emulsion rate (rpm) |
|---|---|---|---|---|---|---|---|---|---|
| | PLGA (mg) | PLGA type | PLGA 5050-PEG 5kD (10% PEG by wt) (mg) | DCM (mL) | Sunitinib malate (mg) | DMSO (mL) | Surfactant | Volume (mL) | |
| | | | | | | | | | |
| JCK--1-72-1 | 400 | 5050 2A | | 8 | 80 | 2 | 1% PVA in H2O | 200 | 4000 |
| YY-1-59-1 | 200 | 7525 4A/ 7525 1.5A (1:1) | 2 | 3 | 40 | 1 | 1% PVA in PBS | 100 | 4000 |
| YY-1-83-1 | 554 | 7525 4A | 6 | 4 | 160 | 2 | 1% PVA in PBS | 200 | 5000 |
| YY-1-83-2 | 504 | 7525 4A | 56 | 4 | 160 | 2 | 1% PVA in PBS | 200 | 5000 |
| YY-1-93-1 | 400 | 7525 4A | 4 | 4 | 90 | 2 | 1% PVA in PBS | 200 | 4000 |
| YY-1-93-2 | 560 | 7525 4A | 5.6 | 4 | 90 | 2 | 1% PVA in PBS | 200 | 5000 |
| YY-1-96-1 | 2240 | 7525 4A | 22.4 | 16 | 360 | 8 | 1% PVA in PBS | 800 | 3000 |
| JCK-1-26-8 | 100 | 7525 6E | | 2 | 75 | 1 | 1% PVA in PBS | 100 | 4000 |

*In vitro* drug release

MPs containing sunitinib (10 mg total weight) were suspended in 4 mL of PBS containing 1% TWEEN® 20 in a 6-mL glass vial and incubated at 37°C under shaking at 150 rpm. At predetermined time points, 3 mL of the supernatant was withdrawn after particles settled to the bottom of the vial and replaced with 3 mL of fresh release medium. The drug content in the supernatant was determined by UV-Vis spectrophotometry or HPLC.

**Results**

Table 8 shows the loading and encapsulation efficiency of MPs prepared according to the formulations in Table 7.

**Table 8: Sunitinib encapsulation efficiency of MPs prepared in Table 6**

| Formulation ID | Drug loading (wt%) | Target loading (wt%) | Encapsulation efficiency (%) |
|---|---|---|---|
| JCK-1-72-1 | 3.4 | 16.7 | 20.1 |
| YY-1-59-1 | 6.8 | 16.5 | 41.2 |
| YY-1-83-1 | 20.5 | 22.2 | 92.2 |
| YY-1-83-2 | 20.2 | 22.2 | 90.7 |
| YY-1-93-1 | 12.4 | 18.2 | 68.1 |
| YY-1-93-2 | 11.6 | 13.7 | 84.5 |
| YY-1-96-1 | 10.1 | 13.7 | 73.6 |
| JCK-1-26-8 | 34.5 | 42.9 | 80.5 |

Figure 2A shows the *in vitro* release profiles ranging from about 1 month to about 6 months of selected MP formulations listed in Table 5 and those listed in Table 7. The PEG-PLGA( PLA) and PEG-PLGA/blend microparticles display sustained release of sunitinib. Sustained release of particles can be tuned as necessary to improve the therapeutic profile by adjusting the ratio of lactide:glycolde in the PLGA copolymer, polymer concentration, drug to polymer ratio, and particle size.

**Example 6: Sunitinib-loaded biodegradable microspheres inhibit experimental corneal neovascularization**

**Introduction**

The cornea, featured by avascularity and transparency, serves as a mechanical barrier and the anterior refractive surface of the eye. Corneal neovascularization (NV) occurs in various pathological conditions including infection, chemical or traumatic injury, autoimmune disease and cornea transplantation, and it can lead to compromised visual acuity if remains untreated. Therefore, the effective inhibition of corneal NV is important to save the vision. Treatments for corneal NV include topical corticosteroids, non-steroid anti-inflammatory medications, and photocoagulation, however, none of the modalities result in permanent cure with some undesirable side effects.

Pathological corneal NV is caused by the disruption of homeostasis between angiogenic and antiangiogenic factors. The vascular endothelial growth factor (VEGF) and platelet-derived growth factor (PDGF) are the key mediators in development of corneal NV. VEGF and its receptors (VEGFR) are present in neovascularized corneas at higher concentrations in comparison to the normal cornea. VEGF play their effects through tyrosine kinase receptors (VEGFR1, 2, 3) leads to signaling for vascular endothelial cell proliferation, migration and survival. VEGF blockade inhibits corneal NV. Sprouting endothelial cells secrete PDGF, and PDGF stimulates VEGF transcription *via* tyrosine kinase PDGF receptors. Pericytes express PDGFR-β, and endothelial cells undergo apoptosis if there are no pericyte support and VEGF signaling. Inhibition of the PDGF signaling pathway disrupts pericyte recruitment and in turn inhibits the angiogenesis.

Anti-VEGF agents including monoclonal antibodies, ribonucleic aptamer and VEGF trap have been applied to prevent and treat corneal NV in the animal studies and clinical trials, which showed limited or partial reduction in pathological corneal NV. The combination of both VEGFR and PDGFR can significantly enhance the efficacy in the antiangiogenesis. Combined inhibition of VEGFRs and PDGFRs with sunitinib was approved to treat gastrointestinal stromal tumor, pancreatic cancer and renal cell carcinoma. Small molecule tyrosine receptor kinase inhibitors (TKI) such as sunitinib, pazopanib and sorafinib targeting against VEGF and PDGF receptors demonstrate features of high potency and efficacy in treating corneal NV.

The topical application of TKI demonstrates efficacy in treating corneal NV in both animal models and clinic trials (Amparo, F., et al., Investigative Ophthalmology & Visual Science, 2013. 54(1): p. 537-544; Cakmak, H., et al., Cutan Ocul Toxicol, 2015: p. 1-7; Perez-Santonja, J.J., et al., Arch Soc Esp Oftalmol, 2013. 88(12): p. 473-81; Ko, B.Y., et al., Cornea, 2013. 32(5): p. 689-95). However, the topical eye drops showed limited bioavailability because of poor drug penetration, rapid tear film turnover and clearance (Govindarajan, B. and I.K. Gipson, Exp Eye Res, 2010. 90(6): p. 655-63; Gaudana, R., et al., Pharm Res, 2009. 26(5): p. 1197-216.) In order to achieve therapeutic effects, the TKI eye drops have to be applied frequently, and in a clinical trial of using TKI pazopanib to treat corneal NV in patients, the eye drops were applied four times per day (Amparo 2013). Frequent administration leads to poor patient compliance.

Biodegradable polymer nano- and microparticles show advantages for the ophthalmic use such as the controlled

drug delivery, enhanced ocular penetration, improved bioavailability and reduced drug side effect (Makadia, H.K. and S.J. Siegel, Polymers (Basel), 2011. 3(3): p. 1377-1397; Shive, M.S. and J.M. Anderson, Adv Drug Deliv Rev, 1997. 28(1): p. 5-24)

Accordingly, a biodegradable polymeric microsphere system for SC administration that can provide sustained release of sunb-malate to effectively inhibit corneal NV was developed and tested in a rat model *in vivo.*

Corneal neovasularization (NV) predisposes patients to compromised corneal transparency and visional acuity. Sunitinib malate (Sunb-malate) targeting against multiple receptor tyrosine kinases, exerts potent antiangiogenesis. However, the rapid clearance of sunb-malate drug administered through topical instillation limits its therapeutic efficacy and poses a challenge for potential patient compliance.

As demonstrated below, sunb-malate-loaded poly(D,L-lactic-co-glycolic acid) (PLGA) microspheres (Sunb-malate MS) with a particle size of approximately 18 μm and a drug loading of 6 wt%. Sunb-malate MS provided sustained the drug release for up to 25 days under the *in vitro* infinite sink condition. Subconjunctival (SC) injection of Sunb-malate MS provided a prolonged ocular drug retention and did not cause ocular toxicity at a dose of 150 μg of active agent. Sunb-malate MS following SC injection more effectively suppressed the suture-induced corneal NV than either sunb-malate free drug or the placebo MS. Local sustained release of sunb-malate through the SC injection of Sunb-malate MS mitigated the proliferation of vascular endothelial cells and the recruitment of mural cells into the cornea. Moreover, the gene upregulation of proangiogenic factors induced by the pathological process was greatly neutralized by SC injection of Sunb-malate MS.

## Materials and Methods

### Materials

Poly(D, L-lactic-co-glycolic acid LA:GA 50:50, MW ~5.6 kDa, acid terminated) (PLGA) was purchased from Lakeshore Biomaterials (Evonik, Birmingham, AL) and sunitinib malate was purchased from LC laboratories (Woburn, MA). Sunb-malate free drug solution was prepared by dissolving sunb-malate in phosphate buffer solution (PBS, pH 7.4) at a concentration of 0.5%. Polyvinyl alcohol (PVA) with MW ~25 kDa was purchased from Polysciences, Inc. (Warrington, PA). Other organic solvents were provided by Sigma-Aldrich (St. Louis, MO).

### Animals

All experimental protocols were approved by the Johns Hopkins Animal Care and Use Committee. 6-8 weeks old male Sprague Dawley rats were purchased from Harlan company (Indianapolis, IN). All rats were cared for and treated in accordance with the Association for Research in Vision and Ophthalmology (ARVO) concerning the use of animals in ophthalmic research. The animals were anesthetized with intramuscular injection of a mixture of ketamine (50 mg/kg) and xylazine (5 mg/kg) during experimental procedures. Topical instillation of 0.5% proparacaine and 0.5% tropicamide were used for topical anesthesia and pupil dilation, respectively.

### Preparation of Sunb-malate loaded PLGA microspheres

Sunb-malate loaded PLGA microspheres (Sunb-malate MS) were prepared using an emulsification method. In brief, 50 mg sunb-malate was dissolved in 0.625 mL dimethyl sulfoxide (DMSO) before mixing with 2.5 mL dichloromethane (DCM) solution containing 250 mg PLGA. The mixture was poured into 60 mL of 1% PVA solution under homogenization at 5000 rpm using a L4RT High Shear mixer (Silverson, East Longmeadow, MA). The formed emulsion was added to an extra 100 mL 0.3% PVA solution under magnetic stirring at 700 rpm for 1 hour. The suspension was placed in a vacuum chamber for another 3 hours under stirring to further remove DCM. The Sunb-malate MS were filtered with 40 μm strainer, washed with DI water and collected by centrifugation at 500× g for 10 minutes. The placebo microspheres (placebo-MS) were prepared with the same procedures without the addition of drug.

### Drug loading and drug release *in vitro*

A determined amount of lyophilized Sunb-malate MS was dissolved in solubilized in DMSO and the solution was measured by UV-Vis at 441 nm on a BioTek Microplate Reader (Winooski, VT). The sunb-malate concentration was calculated using an established standard curve of sunb-malate. The drug loading (DL) and encapsulation efficiency (EE) were calculated as follows:

$$DL\ (\%) = \frac{amount\ of\ \text{sunb} - \text{malate}\ in\ MS}{weight\ of\ MS}$$

$$EE\ (\%) = \frac{actual\ drug\ loading}{theoretical\ drug\ loading}$$

To study the *in vitro* drug release profile of Sunb-malate MS, 1 mL of Sunb-malate MS suspension in PBS (PH 7.4) in a 1.5 mL siliconized Eppendorf tube was shaked at 120 RPM under 37°C. At predetermined time points, the suspension was centrifuged at 2000× g for 5 mins, the supernatant was replaced by 1 mL fresh PBS. The concentration of sunb-malate in the collected supernatant was measured by UV-Vis.

Drug ocular retention *in vivo*

Thirty microliters of Sunb-malate MS or sunb-malate free drug solution at concentration of 5 mg sunb-malate /mL in PBS was administered to rats through SC injection using a 27-gauge needle. At post injection (PI) day 0, 1, 3, 7, 14 and 28, the whole eyeballs (n=4) were harvested after the animals were sacrificed. Sunb-malate exhibits autofluorescence, therefore the enucleated eyeballs were imaged using the Xenogen IVIS Spectrum optical imaging system (Caliper Life Sciences Inc., Hopkinton, MA) at the excitation and emission wavelength of 420 nm and 510 nm, respectively. The fluorescent images were analyzed using the Living Image 3.0 software (Caliper Lifesciences, Inc.), and the retention of sunitinib was quantified by comparing to the fluorescence counts of the eye immediately undertaken SC injection. Rat eyes without SC injection were used as the baseline.

*In vivo* safety studies

In order to determine the ocular toxicity of Sunb-malate MS following SC injection, 30 μL Sunb-malate MS at concentrations of 5 and 0.5 mg Sunb-malate /mL, were administered to both eyes of Sprague Dawley rats. The SC injection of saline and placebo MS (2.5 mg particles per eye) were used as control. At both PI day 7 and day 14, two animals were sacrificed to harvest the whole eyeballs with conjunctiva tissue for histological examination. The injection site was marked with a 6-0 Nylon suture. The eyeballs were fixed in formalin, embedded in paraffin, sectioned along the anteroposterior axis (from cornea to optic nerve) to cut through the SC injection site, and stained with the hematoxylin and eosin (H&E). The slides were observed and graded by a pathologist.

The treatment of corneal NV

Corneal NV was induced by intrastromal suturing. In brief, two intrastromal suture stitches were placed in the superior cornea with 10-0 nylon (Alcon Laboratories, Inc, Fort Worth, TX) under an operating microscope after rats were anesthetized and pupil dilated. The distance between the stitch and the limbus was approximately 2 mm while there was a distance of 1 mm between the two stitches. After suturing, animals were immediately treated with a single SC injection of 30 μL of (1) PBS, (2) placebo-MP, (3) Sunb-malate MS (5 mg sunb-malate /mL), and (4) Sunb-malate free drug solution (5 mg sunb-malate /mL). Erythromycin antibiotic ointment was applied to prevent potential infection and corneal dry-up. The rats were followed for 2 weeks.

Quantitative analysis of corneal NV

The corneas of all rats were examined by slit-lamp biomicroscope (SL120; Carl Zeiss AG, Oberkochen, Germany) and corneal photographs were taken with a digital camera. The area and length of vascularized cornea were quantified with Photoshop CS3.0. The arc was drawn along the limbus, the vascularized area pixel measured and the corneal NV area was calculated using the following equation:

$$Corneal\ NV\ area = \frac{\text{pixel of vascularized area}}{pixel\ to\ occupy\ 1\ mm^2\ area}$$

The vascularized area was evenly divided into six sections. The distance between vessel tips and the limbus at the five intersection points of the arc was measured. The average of the five measured lengths was regarded as the corneal NV length.

Real-time quantitative reverse transcription-polymerase chain reaction (RT-PCR)

At post-operative (PO) day 7 and day 14, rats were sacrificed and the corneas were collected. Three corneas at the same condition were pooled together for mRNA isolation. Total mRNA was isolated with TRIzol® reagent (Invitrogen, USA) according to the manufacturer's instructions, followed by reverse transcription using the High Capacity cDNA Reverse Transcription Kit (Applied Biosystems, USA). To quantify the mRNA expression levels of angiogenic and anti-angiogenic factors including VEGF, VEGFR1, VEGFR2, PDGFa, PDGFb, PDGFRα, PDGFRβ, VE-cadherin, Ang1, MMP2, MMP9, bFGF and PECAM1, RT-PCR was performed with Fast SYBR® Green Master Mix using a 7100 Real Time PCR System (Applied Biosystems, CA). The mRNA expression levels were normalized to GAPDH for further multiple group comparison.

Immunostaining and confocal imaging

The eyeballs were enucleated and fixed with 4% paraformaldehyde (PFA) for 1 h at 4 °C. Subsequently, the corneas were dissected, washed with PBS, cryoprotected in 15% sucrose PBS solution, and embedded in OCT compound. Serial corneal sections (30 μm in thickness) were collected by cryostat sectioning, followed by immunostaining using the following antibodies: mouse platelet endothelial cell adhesion molecule-1 (PECAM-1, 1:500; Abcam), rabbit neural/glial antigen-2 (NG2, 1;500; Millipore), donkey anti-mouse Cy2 and donkey anti-rabbit Cy3 diluted in PBS containing 10% donkey serum and 0.1% Triton X-100. After overnight incubation at 4°C, the corneal sections were washed for three times in PBS and incubated with secondary antibody at room temperature for 2 h. The mounted corneal sections were imaged using Zeiss LSM 710 confocal microscopy (Carl Zeiss, Germany). To secure the panoramic images of cornea, we serially aligned the corneal sections along the anteroposterior axis using Reconstruct 1.1.0 (J.C. Fiala, NIH) and performed a maximum-intensity projection.

Statistical analysis

The quantitative results were presented as the average of multiple repeats ± standard error of the mean (SEM). All data collected were compared among groups using t test and multiple comparisons test (one-way ANOVA, Bonferroni test). Differences were considered to be statistically significant at a level of $P < 0.05$. Significance for multiple comparisons: *$P < 0.05$; **$P < 0.01$; ***$P < 0.001$.

**Results**

Preparation and characterization of Sunb-malate MS

Sunb-malate MS were porous by SEM with a particle size of 15±9 μm and a surface charge of -0.7 mV (Table 4). Sunb-malate MS exhibited a drug loading of 6 wt%, and sunb-malate was released in a steady fashion up to 25 days under the infinite sink condition at 37 °C *in vitro* without an obvious initial rapid drug release phase (Figure 3).

**Table 4. Physiochemical characteristics of PLGA microspheres**

| | Size (μm) | Surface charge (mV) | Drug loading |
|---|---|---|---|
| Sunb-malate MS | 15±9 | -1.1 ± 0.2 | 7% |
| Placebo-MS | 13±5 | -0.7 ± 0.2 | N/A |

Ocular drug retention

Sunb-malate free drug was quickly cleared within 1 day after SC injection. As shown in Figure 4, the encapsulation of sunb-malate into PLGA MS significantly prolonged the ocular drug retention and approximately 50% of original drug was retained by PI day 14, and the sunb-malate was gradually disappeared by PI day 28.

Ocular safety of Sunb-malate MS

In order to determine the ocular safety after the SC injection of placebo MS and the Sunb-malate MS, we carried out the histological examination of the eyes. SC injection of placebo MS is safe as SC injection of saline. Both the low dose and high dose of Sunb-malate MS (0.5 and 5 mg sunb-malate/ml) following SC injection did not induce inflammation in the conjunctiva tissue at the injection site and the cornea.

Effect of Sunb-malate MS on corneal NV

All animals were examined by slit-lamp biomicroscopy at post-operative day (POD) 5, 7 and 14 to evaluate the corneal NV under the treatment of Sunb-malate MS, Sunb-malate free drug and placebo-MP. Radially-oriented new blood vessels invaded into the cornea from the limbus toward the suture stitches by POD 5 and further grew to reach the stitches by POD 14 for placebo MS treated rats. SC injection of Sunb-malate free drug showed negligible effect on the inhibition of corneal NV, although there was a slight inhibition of corneal NV area as compared with the placebo MS at POD 5, but not statistically significant. In contrast, the quantification of corneal NV length (Figure 5A) and area (Figure 5B) revealed that Sunb-malate MS significantly suppressed the sprouting of new blood vessel at POD 5 and stalled the further growth over time. The histopathological analysis further confirmed that the ingrowth of new blood vessels which were observed in the Sunb-malate and placebo-MS treated corneas were strikingly mitigated by SC injection of Sunb-malate MS at both POD 7 and 14. Corneal inflammation and corneal edema were not observed under the treatment of SC injection of 5 mg/ml Sunb-malate MS. Therefore, Sunb-malate MS provided a safe and efficient inhibition against corneal angiogenesis.

Sunb-malate MS downregulated the mRNA expression levels of angiogenic effectors

The mRNA expression of angiogenic factors, endothelial cell markers and matrix metalloproteases was determined by RT-PCR at POD 7 and 14. The quantitative analysis at POD 7 showed that the expression of VEGF, VEGFR1, PDGFb, PDGFRs, VE-cadherin, bFGF, MMPs and Ang1 in the cornea was significantly decreased by SC injection of Sunb-malate MS as compared to placebo-MP and Sunb-malate free drug, although there was no statistically significant difference in the mRNA levels of VEGFR2 and PDGFa between Sunb-malate MS and Sunb-malate free drug treatment groups (Figures 6A-6M). A similar result at POD 14 indicated a sustainable suppression of angiogenesis-associated gene expression with SC injection of Sunb-malate MS.

Sunb-malate MS suppressed the mural cell recruitment

To investigate the effect of SC injection of Sunb-malate MS on the ingrowth of vascular endothelial cells and the subsequent recruitment of vascular mural cells including pericytes around capillaries and smooth muscle cells around larger vessels, corneas were collected following SC injection of Sunb-malate MS for immunohistochemical analysis. The panoramic images of cornea showed that the growth of corneal vasculature was conspicuously suppressed by SC injection of Sunb-malate MS when compared with SC injection of placebo MS. The recruitment of NG2-positive mural cells was more mitigated than the PECAM-positive endothelial cells by SC injection of Sunb-malate MS.

In summary, sunb-malate encapsulated into biodegradable PLGA MS provided an efficient inhibition of corneal NV in a suture-induced corneal NV model. Sunb-malate can be dissolved in PBS at concentrations of at least 25 mg/mL. Almost observed no drug retention in the eye is observed within 24 hours following the SC injection of sunb-malate free drug solution to rats. Through the encapsulation of water-soluble sunb-malate into biodegradable PLGA MS, significantly longer retention of sunb-malate was observed, up to 28 days with 50% at PI day 14. Particles can be constantly retained in the injection site following the initial injection and particle leakage. The enhanced retention of particles following SC injection and sustained drug release contributed to prolonged drug retention in the injection site. The efficacy of SC injection of Sunb-malate MS in inhibiting corneal NV are resulted from two important factors: (1) successful encapsulation of water-soluble sunb-malate into biodegradable polymeric MS and (2) the efficient intraocular penetration of the water-soluble sunb-malate released from the Sunb-malate MS following the SC injection.

The results demonstrated that the upregulation of endothelial cell markers (VE-cadherin and PECAM1), metalloproteinases (MMP2 and MMP9), proangiogenic factors and their receptors (VEGF, PDGFs, bFGF, Ang1, VEGFRs and PDGFRs) are largely abolished by the administration of Sunb-malate MS. MMPs participate in the degradation of extracellular matrix and the remodeling of vascular basement membrane, which are required for angiogenesis. VEGF, PDGFs, bFGF and Ang1 were involved in regulating angiogenesis by binding and activating the corresponding receptor tyrosine kinase on the cell surface. In the present study, the gene expression of many pro-angiogenic factors and angiogenesis-associated protease is downregulated by the Sunb-malate MS in the suture-induced animal model, demonstrating its anti-angiogenic activities at the molecular level.

Biocompatible and biodegradable PLGA microspheres allowed a sustained release of sunb-malate and a prolonged retention of drug on ocular surface. The safe dose of Sunb-malate MS was determined by concentration-gradient analysis in the animal models. SC injection of Sunb-malate MS significantly inhibited the corneal NV in the suture-induced model. Together, the sustained release of sunb-malate by SC injection of Sunb-malate MS could improve the efficacy, reduces the toxicity and overcomes the non-compliance of patients. The study provides a therapeutic strategy targeting against corneal NV.

**Example 7: Persistance and Biocompatibility of Sunitinib Particles Materials and Methods**

Cohorts of C57BL/6 mice (n=5) had IVT injection of sunitinib microparticles (10 μg total drug content) and laser-induced disruption of Bruch's membrane at 0, 2, 4, or 8 weeks after injection. The area of CNV was measured one week after laser treatment (i.e., weeks 1, 3, 5, and 9). Immediately after or 2, 4, or 8 weeks later, mice (n=5) were subjected to laser disruption of Bruch's membrane, and one week later the size of the CNV lesions was quantitated.

A pharmacokinetic study was also conducted using normal C57BL/6 mice and the drug levels in different ocular tissues were determined by HPLC-MS at various time points following IVT injection of the microparticles. Fundus images were taken at1, 2, and 3 months after a single injection of sunitinib-releasing microparticles.

Histological images of the retina in rabbit eyes 3 months after injection of either phosphate buffered saline or sunitinib-releasing microparticles was used to measure the inflammatory response.

**Results**

The microparticles had a drug loading of 3.4% (by weight) and a mean diameter of about 13 μm. A significant reduction in the area of the CNV was observed in all animals treated with the sunitinib microparticles compared to the controls. Importantly, the protective effect was sustained for at least 9 weeks following IVT injection of the microparticles.

Sunitinib-releasing microparticles were retained and provided sustained sunitinib levels for at least 3 months in the rabbit eye *in vivo*.

Sunitinib has a characteristic yellow color that was apparent at the injection site for at least 3 months. The average sunitinib concentration in the vitreous of these rabbits at 3 months was 1.6 μM, which is in the target range for maximal RGC survival based on *in vitro* culture. The overall concentration of sunitinib in the microparticles clearly decreased as the drug was released into the vitreous over time, indicated by the decrease in the intensity of the yellow color. The average concentration of sunitinib in the vitreous at 3 months was found by HPLC-MS to be 1.6 μM, which falls in the target range for maximal RGC survival based on *in vitro* primary RGC culture methods.

Histological images of the rabbit eyes obtained at 3 months after injection of the sunitinib-releasing microparticles were analyzed by the Director of Ophthalmic Pathology, Dr. Charles Eberhart. No inflammatory response was observed in half of the eyes, and mild inflammation around the microparticle aggregate was observed in half of the eyes.

Increasing the PEG content of the sunitinib-releasing microparticles, should further minimize potential inflammatory responses while maintaining therapeutic sunitinib levels in the eye for at least 6 months with a single injection.

**Example 8: Treatment of Age Related Macular Degeneration**

Age related macular degeneration (AMD) is a leading cause of severe, irreversible vision loss among the elderly. Bressler, et al. JAMA, 291:1900-1901(2004). AMD is characterized by a broad spectrum of clinical and pathologic findings, such as pale yellow spots known as drusen, disruption of the retinal pigment epithelium (RPE), choroidal neovascularization (CNV), and disciform macular degeneration. AMD is classified as either dry (*i.e.,* non-exudative) or wet (*i.e.,* exudative). Dry AMD is characterized by the presence of lesions called drusen. Wet AMD is characterized by neovascularization in the center of the visual field.

Although less common, wet AMD is responsible for 80%-90% of the severe visual loss associated with AMD (Ferris, et al. Arch. Ophthamol. 102:1640-2 (1984)). The cause of AMD is unknown. However, it is clear that the risk of developing AMD increases with advancing age. AMD has also been linked to risk factors including family history, cigarette smoking, oxidative stress, diabetes, alcohol intake, and sunlight exposure.

Wet AMD is typically characterized by CNV of the macular region. The choroidal capillaries proliferate and penetrate Bruch's membrane to reach the retinal pigment epithelium (RPE). In some cases, the capillaries may extend into the subretinal space. The increased permeability of the newly formed capillaries leads to accumulation of serous fluid or blood under the RPE and/or under or within the neurosensory retina. Decreases in vision occur when the fovea becomes swollen or detached. Fibrous metaplasia and organization may ensue, resulting in an elevated subretinal mass called a disciform scar that constitutes end-stage AMD and is associated with permanent vision loss (D'Amico D J. N. Engl. J. Med. 331:95-106 (1994)).

Sustained suppression of murine choroidal neovascularization by intravitreous injection of sunitinib-encapsulated polymer microparticles has now been demonstrated. The long-term efficacy of sunitinib released from biodegradable polymer microparticles following intravitreous (IVT) injection in a mouse model of laser-induced choroidal neovascularization was demonstrated as follows.

**Materials and Methods**

Biodegradable polymer microparticles were prepared for sustained delivery of sunitinib were prepared as described in the foregoing examples. Microparticles were characterized *in vitro,* including average size, size distribution, drug loading, and drug release profile.

Materials and Methods

Pathogen-free C57BL/6 mice (Charles River, Wilmington, MA) were treated in accordance with the Association for Research in Vision and Ophthalmology Statement for the Use of Animals in Ophthalmic and Vision Research and the guidelines of the Johns Hopkins University Animal Care and Use Committee.

Choroidal NV was induced by laser photocoagulation-induced rupture of Bruch's membrane as previously described (Tobe, T. et al., Am. J. Pathol. 135(5): 1641-1646(1998)). Briefly, 5-6-week-old female C57BL/6 mice were anesthetized with ketamine hydrochloride (100 mg/kg body weight) and pupils were dilated. Laser photocoagulation (75 μm spot size, 0.1 sec duration, 120 mW) was performed in the 9, 12, and 3 o'clock positions of the posterior pole of each eye with the slit lamp delivery system of an OcuLight GL diode laser (Iridex, Mountain View, CA) and a handheld cover slip as a contact lens to view the retina. Production of a bubble at the time of laser, which indicates rupture of Bruch's membrane, is an important factor in obtaining choroidal NV; therefore, only burns in which a bubble was produced were included in the study.

Immediately after laser-induced rupture of Bruch's membrane, mice were randomized to various treatment groups for intraocular injections. Intravitreal injections were done under a dissecting microscope with a Harvard Pump Microinjection System and pulled glass micropipettes.

Cohorts of C57BL/6 mice (n=5) had intravitreal (IVT) injection of sunitinib microparticles (10 μg total drug content) and laser-induced disruption of Bruch's membrane at 0, 2, 4, or 8 weeks after injection. The area of CNV was measured one week after laser treatment (i.e., weeks 1, 3, 5, and 9).

A pharmacokinetic study was also conducted using normal C57BL/6 mice and the drug levels in different ocular tissues were determined by HPLC-MS at various time points following IVT injection of the microparticles.

**Results**

The microparticles had a drug loading of 3.4% (by weight) and a mean diameter of about 13 μm.

A significant reduction in the area of the CNV was observed in all animals treated with the sunitinib microparticles compared to the controls, as shown in Figures 7A-7D. Importantly, the protective effect was sustained for at least 9 weeks following IVT injection of the microparticles.

Modifications and variations of the sunitinib formulations and methods of use thereof will be apparent to those of skill in the art and are intended to come within the scope of the appended claims.

## Claims

**1.** Polymeric microparticles comprising sunitinib or a pharmaceutically acceptable salt thereof encapsulated or dispersed in a blend of (a) polylactic acid and/or a copolymer of lactic acid and glycolic acid and (b) polylactic acid conjugated to a polyalkylene oxide and/or a copolymer of lactic acid and glycolic acid conjugated to a polyalkylene oxide, wherein the sunitinib or a pharmaceutically acceptable salt thereof is present in a weight loading of greater than 5% and wherein the microparticles provide sustained release of sunitinib or the pharmaceutically acceptable salt thereof.

**2.** The polymeric microparticles of claim 1, wherein the sunitinib is present in the form of a malate salt.

**3.** The polymeric microparticles of any of claims 1 or 2 wherein the blend is a blend of poly(lactide-co-glycolide) (PLGA) and PLGA conjugated to polyethylene glycol (PEG).

**4.** The polymeric microparticles of any of claims 1-3, wherein the microparticles have a weight loading of 10% or greater by weight sunitinib or the pharmaceutically acceptable salt thereof.

**5.** The polymeric microparticles of any claims 1-4 wherein the microparticles have an average diameter between one and 50 microns.

**6.** The polymeric microparticles of any one of claims 1-5, wherein the PLGA and PLGA-PEG are present in a mixture from 50% PLGA and 50% PLGA-PEG to 99% PLGA and 1% PLGA-PEG by weight.

7. The microparticles of claim 3, wherein the PLGA and PLGA-PEG are present in a mixture of 99% PLGA and 1% PLGA-PEG.

8. The polymeric microparticles of any of claims 3, 6 or 7, wherein the PLGA-PEG is formed from PLGA with a molecular weight of 45 KDa and PEG with a molecular weight of 5 KDa.

9. The polymeric microparticles of claims 1 or 2 wherein the blend is a blend of polylactic acid (PLA), poly(lactide-co-glycolide) (PLGA) and PLGA conjugated to polyethylene glycol (PEG).

10. A pharmaceutical composition for ocular delivery comprising an effective amount of the microparticles of any of claims 1-9 in a pharmaceutically acceptable carrier.

11. The pharmaceutical composition of claim 10, wherein the sunitinib is is present in the form of a malate salt.

12. The pharmaceutical composition of any of claims 10 or 11 comprising microparticles having an average diameter of between one and 30 microns.

13. The pharmaceutical composition of any of claims 10-12 wherein sunitinib or its pharmaceutically acceptable salt is encapsulated in microparticles in an amount of at least 10% by weight.

14. The pharmaceutical composition of claim 13, wherein sunitinib or its pharmaceutically acceptable salt is encapsulated in microparticles in an amount of at least 15% by weight.

15. The pharmaceutical composition of any of claims 10-12 wherein the PLGA and PLGA-PEG are present in a mixture of about 99% PLGA and 1% PLGA-PEG by weight.

16. The pharmaceutical composition of any of claims 10-14 wherein the PLGA and PLGA-PEG are present in a mixture from 50% PLGA and 50% PLGA-PEG to 99% PLGA and 1% PLGA-PEG by weight.

17. The pharmaceutical composition of claim 16, wherein the PLGA-PEG copolymer has PLGA with a molecular weight of 45 KDa and PEG with a molecular weight of 5 KDa.

18. The composition of any of claims 10-17 wherein the microparticles release sunitinib over a sustained period of at least three months in the vitreous chamber of the eye.

19. The pharmaceutical composition of any of claims 10-18 comprising microparticles having an average diameter of between one and 50 microns.

20. The pharmaceutical composition of any one of claims 10-19 for intravitreal injection.

**Patentansprüche**

1. Polymere Mikropartikel, umfassend Sunitinib oder ein pharmazeutisch annehmbares Salz davon, eingekapselt oder dispergiert in einem Gemisch aus (a) Polylactid und/oder einem Copolymer aus Milchsäure und Glykolsäure und (b) Polylactid, konjugiert mit einem Polyalkylenoxid und/oder einem Copolymer von Milchsäure und Glykolsäure, konjugiert an ein Polyalkylenoxid, wobei Sunitinib oder ein pharmazeutisch annehmbares Salz davon in einer Gewichtsbeladung von über 5 % vorliegt und wobei die Mikropartikel eine verzögerte Freisetzung von Sunitinib oder dem pharmazeutisch annehmbaren Salz davon bewirken.

2. Polymere Mikropartikel nach Anspruch 1, wobei das Sunitinib in Form eines Malatsalzes vorliegt.

3. Polymere Mikropartikel nach einem der Ansprüche 1 oder 2, wobei das Gemisch ein Gemisch aus Polylactid-co-Glycolid (PLGA) und PLGA konjugiert mit Polyethylenglycol (PEG) ist.

4. Polymere Mikropartikel nach einem der Ansprüche 1-3, wobei die Mikropartikel eine Gewichtsbeladung von mindestens 10 Gew.-% Sunitinib oder dem pharmazeutisch annehmbaren Salz davon aufweisen.

5. Polymere Mikropartikel nach einem der Ansprüche 1-4, wobei die Mikropartikel einen durchschnittlichen Durchmesser von zwischen einem und 50 μm aufweisen.

6. Polymere Mikropartikel nach einem der Ansprüche 1-5, wobei PLGA und PLGA-PEG in einem Gemisch von 50 Gew-% PLGA und 50 Gew-% PLGA-PEG bis 99 Gew-% PLGA und 1 Gew-% PLGA-PEG vorliegen.

7. Mikropartikel nach Anspruch 3, wobei PLGA und PLGA-PEG in einem Gemisch aus 99 % PLGA und 1 % PLGA-PEG vorliegen.

8. Polymere Mikropartikel nach einem der Ansprüche 3, 6 oder 7, wobei das PLGA-PEG ausgebildet ist aus PLGA mit einem Molekülgewicht von 45 kDa und PEG mit einem Molekülgewicht von 5 kDa.

9. Polymere Mikropartikel nach Anspruch 1 oder 2, wobei das Gemisch ein Gemisch aus Polylactid (PLA), Polylactidco-Glycolid (PLGA) und PLGA konjugiert mit Polyethylenglycol (PEG) ist.

10. Pharmazeutische Zusammensetzung zur okularen Verabreichung, umfassend eine wirksame Menge der Mikropartikel nach einem der Ansprüche 1-9 in einem pharmazeutisch annehmbaren Träger.

11. Pharmazeutische Zusammensetzung nach Anspruch 10, wobei Sunitinib in Form eines Malatsalzes vorliegt.

12. Pharmazeutische Zusammensetzung nach einem der Ansprüche 10 oder 11, umfassend Mikropartikel mit einem durchschnittlichen Durchmesser von zwischen einem und 30 μm.

13. Pharmazeutische Zusammensetzung nach einem der Ansprüche 10-12, wobei Sunitinib oder sein pharmazeutisch annehmbares Salz in einer Menge von mindestens 10 Gew.-% in Mikropartikeln eingekapselt ist.

14. Pharmazeutische Zusammensetzung nach Anspruch 13, wobei Sunitinib oder sein pharmazeutisch annehmbares Salz in einer Menge von mindestens 15 Gew.-% in Mikropartikeln eingekapselt ist.

15. Pharmazeutische Zusammensetzung nach einem der Ansprüche 10-12, wobei PLGA und PLGA-PEG in einem Gemisch von etwa 99 Gew-% PLGA und 1 Gew-% PLGA-PEG vorliegen.

16. Pharmazeutische Zusammensetzung nach einem der Ansprüche 10-14, wobei PLGA und PLGA-PEG in einem Gemisch von 50 Gew-% PLGA und 50 Gew-% PLGA-PEG bis 99 Gew-% PLGA und 1 Gew-% PLGA-PEG vorliegen.

17. Pharmazeutische Zusammensetzung nach Anspruch 16, wobei das PLGA-PEG-Copolymer PLGA mit einem Molekülgewicht von 45 kDa und PEG mit einem Molekülgewicht von 5 kDa aufweist.

18. Zusammensetzung nach einem der Ansprüche 10-17, wobei die Mikropartikel Sunitinib über einen anhaltenden Zeitraum von mindestens drei Monaten in der Glaskörperkammer des Auges freisetzen.

19. Pharmazeutische Zusammensetzung nach einem der Ansprüche 10-18, umfassend Mikropartikel mit einem durchschnittlichen Durchmesser von zwischen einem und 50 μm.

20. Pharmazeutische Zusammensetzung nach einem der Ansprüche 10-19 für intravitreale Injektion.

**Revendications**

1. Microparticules polymères comprenant du sunitinib ou un sel pharmaceutiquement acceptable de celui-ci encapsulé ou dispersé dans un mélange (a) d'acide polylactique et/ou d'un copolymère d'acide lactique et d'acide glycolique et (b) d'acide polylactique conjugué à un polyoxyde d'alkylène et/ou d'un copolymère d'acide lactique et d'acide glycolique conjugués à un polyoxyde d'alkylène, dans lesquelles le sunitinib ou un sel pharmaceutiquement acceptable de celui-ci est présent à une charge en poids supérieure à 5 % et dans lesquelles les microparticules fournissent une libération prolongée de sunitinib ou du sel pharmaceutiquement acceptable de celui-ci.

2. Microparticules polymères selon la revendication 1, dans lesquelles le sunitinib est présent sous la forme d'un sel de malate.

3. Microparticules polymères selon l'une quelconque des revendications 1 ou 2, dans lesquelles le mélange est un mélange de poly(lactide-co-glycolide) (PLGA) et de PLGA conjugué à du polyéthylène glycol (PEG).

4. Microparticules polymères selon l'une quelconque des revendications 1 à 3, dans lesquelles les microparticules ont une charge en poids de 10 % en poids ou plus de sunitinib ou du sel pharmaceutiquement acceptable de celui-ci.

5. Microparticules polymères selon l'une quelconque des revendications 1 à 4, dans lesquelles les microparticules présentent un diamètre moyen compris entre un et 50 microns.

6. Microparticules polymères selon l'une quelconque des revendications 1 à 5, dans lesquelles le PLGA et le PLGA-PEG sont présents dans un mélange de 50 % de PLGA et 50 % de PLGA-PEG à 99 % de PLGA et 1 % de PLGA-PEG en poids.

7. Microparticules selon la revendication 3, dans lesquelles le PLGA et le PLGA-PEG sont présents dans un mélange de 99 % de PLGA et 1 % de PLGA-PEG.

8. Microparticules polymères selon l'une quelconque des revendications 3, 6 ou 7, dans lesquelles le PLGA-PEG est formé à partir de PLGA d'une masse moléculaire de 45 Kda et de PEG d'une masse moléculaire de 5 KDa.

9. Microparticules polymères selon les revendications 1 ou 2 dans lesquelles le mélange est un mélange d'acide polylactique (PLA), de poly(lactide-co-glycolide) (PLGA) et de PLGA conjugué au polyéthylène glycol (PEG).

10. Composition pharmaceutique pour administration oculaire comprenant une quantité active des microparticules selon l'une quelconque des revendications 1 à 9 dans un véhicule pharmaceutiquement acceptable.

11. Composition pharmaceutique selon la revendication 10, dans laquelle le sunitinib est présent sous la forme d'un sel de malate.

12. Composition pharmaceutique selon l'une quelconque des revendications 10 ou 11, comprenant des microparticules présentant un diamètre moyen compris entre un et 30 microns.

13. Composition pharmaceutique selon l'une quelconque des revendications 10 à 12, dans laquelle le sunitinib ou son sel pharmaceutiquement acceptable est encapsulé dans des microparticules dans une proportion d'au moins 10 % en poids.

14. Composition pharmaceutique selon la revendication 13, dans laquelle le sunitinib ou son sel pharmaceutiquement acceptable est encapsulé dans des microparticules dans une proportion d'au moins 15 % en poids.

15. Composition pharmaceutique selon l'une quelconque des revendications 10 à 12 dans lesquelles le PLGA et le PLGA-PEG sont présents dans un mélange de 99 % de PLGA et 1 % de PLGA-PEG en poids.

16. Composition pharmaceutique selon l'une quelconque des revendications 10 à 14 dans laquelle le PLGA et le PLGA-PEG sont présents dans un mélange de 50 % de PLGA et 50 % de PLGA-PEG à 99 % de PLGA et 1 % de PLGA-PEG en poids.

17. Composition pharmaceutique selon la revendication 16, dans laquelle le copolymère PLGA-PEG contient du PLGA d'une masse moléculaire de 45 Kda et du PEG d'une masse moléculaire de 5 KDa.

18. Composition selon l'une quelconque des revendications 10 à 17 dans laquelle les microparticules libèrent du sunitinib sur une période prolongée d'au moins trois mois dans la cavité vitréenne de l'œil.

19. Composition pharmaceutique selon l'une quelconque des revendications 10 à 18, comprenant des microparticules présentant un diamètre moyen compris entre un et 50 microns.

20. Composition pharmaceutique selon l'une quelconque des revendications 10 à 19 pour injection intravitréenne.

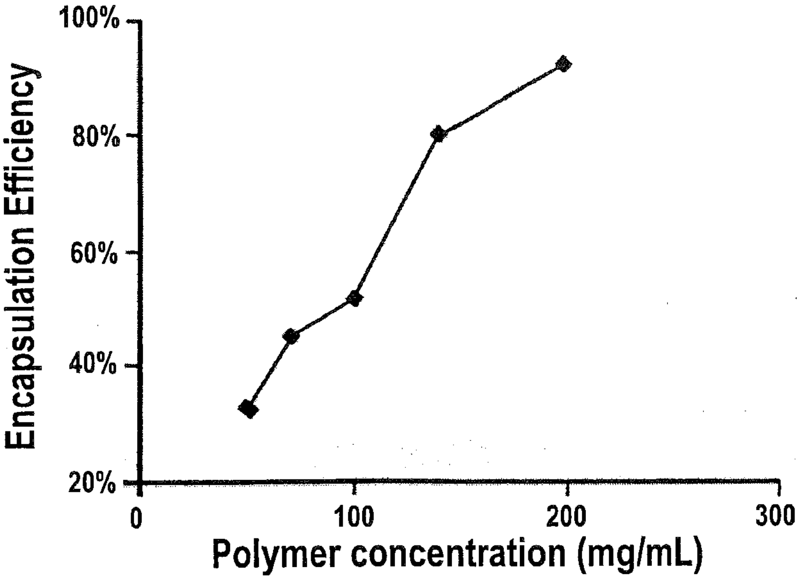
100%
80%
60%
40%
20%
Encapsulation Efficiency
0
100
200
300
Polymer concentration (mg/mL)

*FIG. 1*

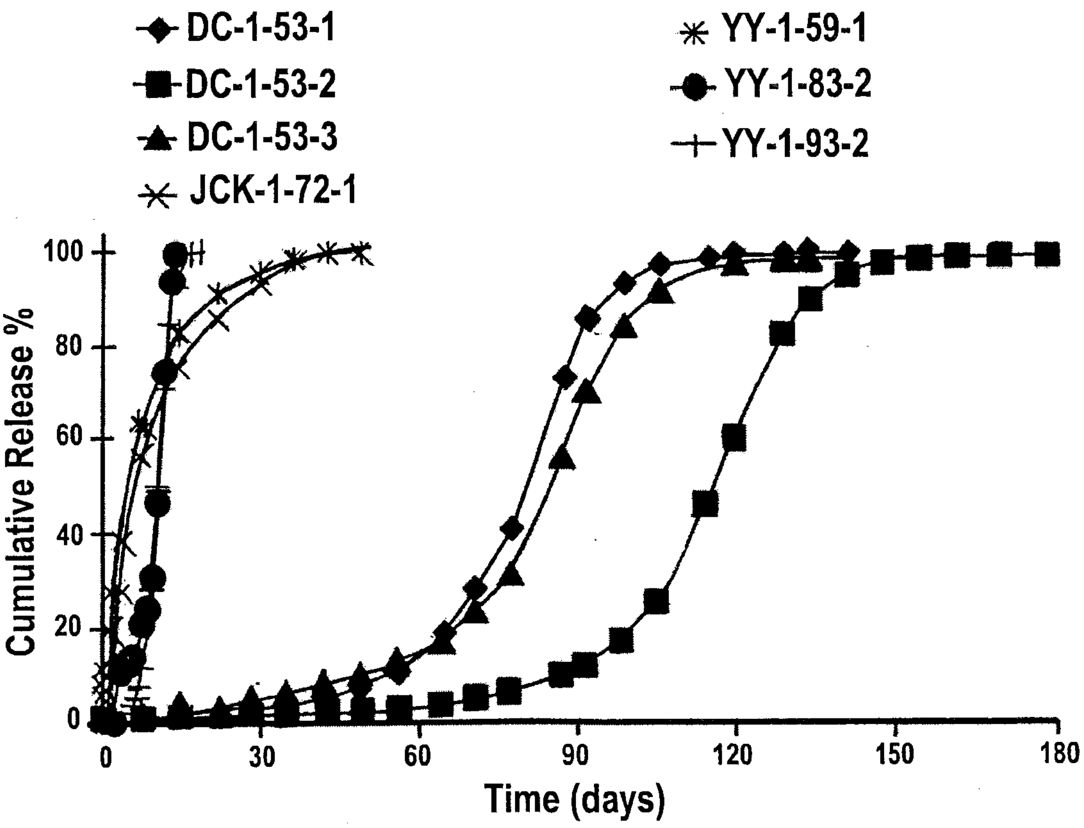
DC-1-53-1
DC-1-53-2
DC-1-53-3
JCK-1-72-1
YY-1-59-1
YY-1-83-2
YY-1-93-2
100
80
60
40
20
0
Cumulative Release %
0
30
60
90
120
150
180
Time (days)

*FIG. 2A*

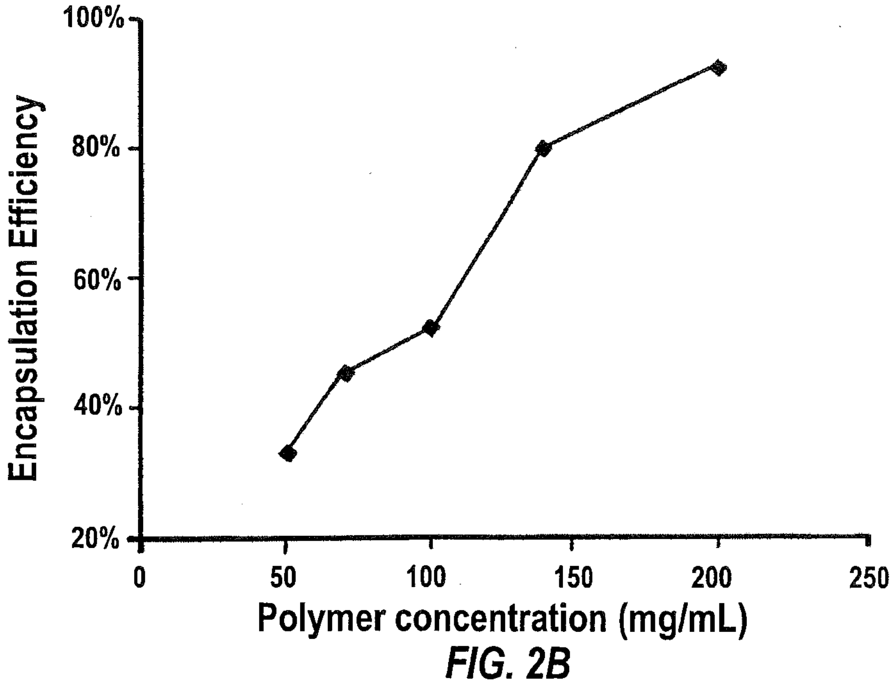
100%
80%
60%
40%
20%
Encapsulation Efficiency
0
50
100
150
200
250
Polymer concentration (mg/mL)

*FIG. 2B*

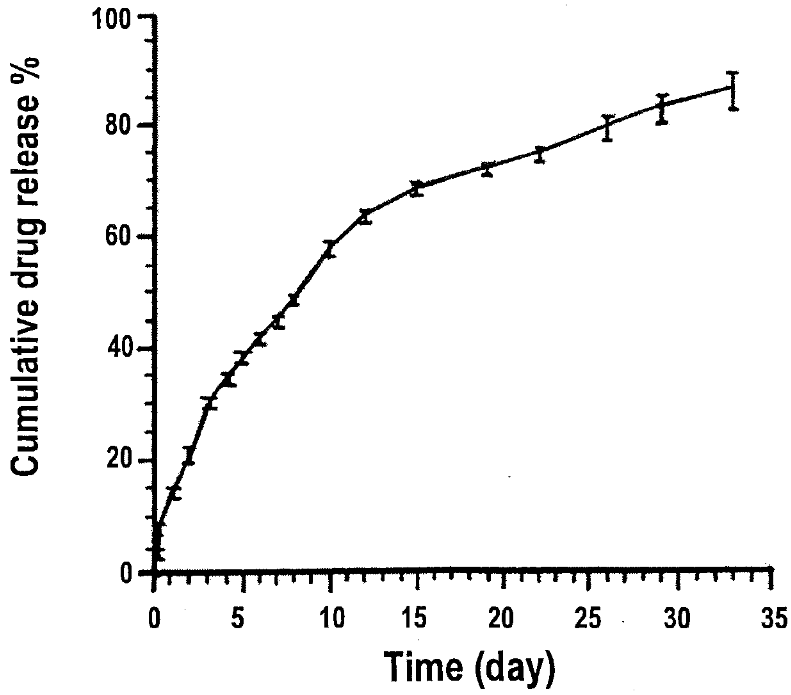
100
80
60
40
20
0
Cumulative drug release %
0
5
10
15
20
25
30
35
Time (day)

*FIG. 3*

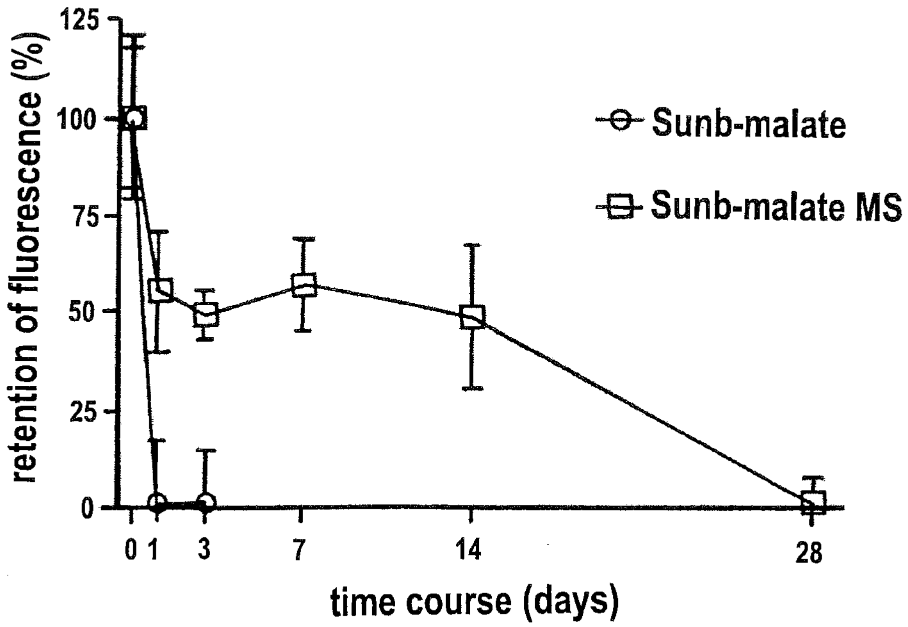
retention of fluorescence (%)
125
100
75
50
25
0
0 1 3 7 14 28
time course (days)
Sunb-malate
Sunb-malate MS

*FIG. 4*

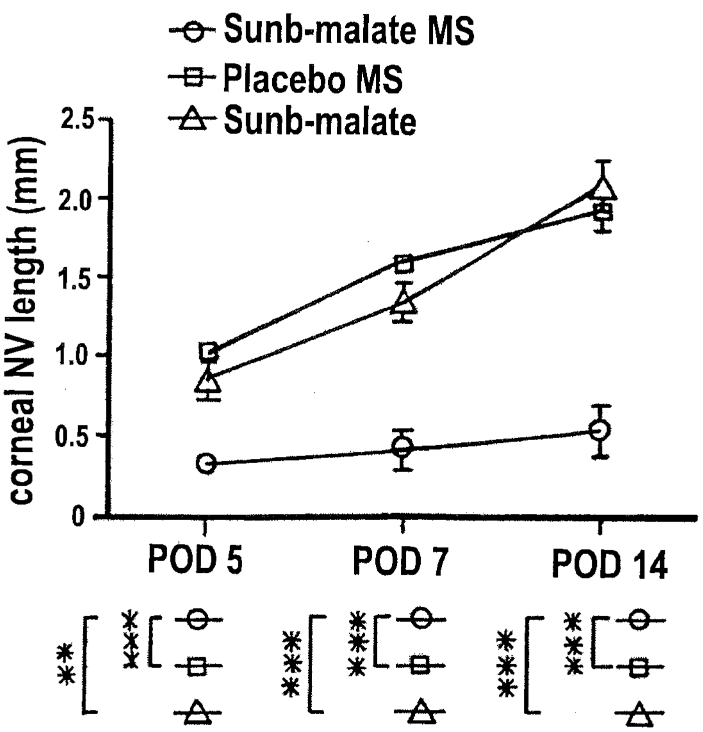
Sunb-malate MS
Placebo MS
Sunb-malate
corneal NV length (mm)
2.5
2.0
1.5
1.0
0.5
0
POD 5
POD 7
POD 14
**
***
***
***
***
***
***
***

*FIG. 5A*

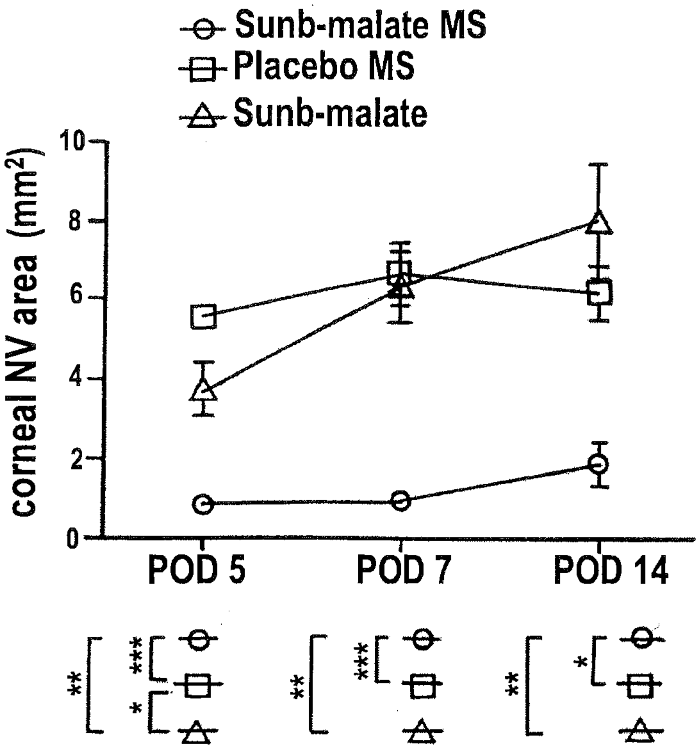


FIG. 5B

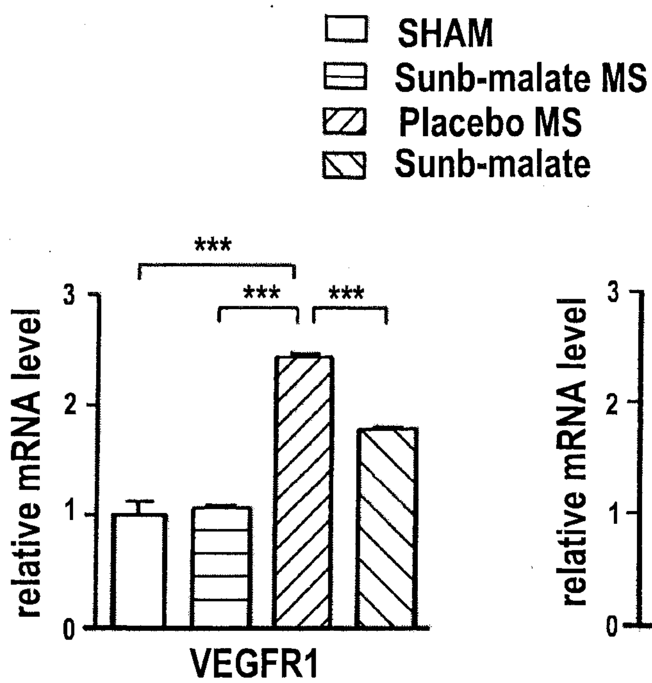


FIG. 6A

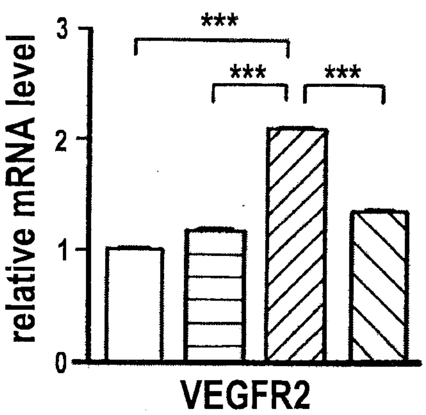


FIG. 6B

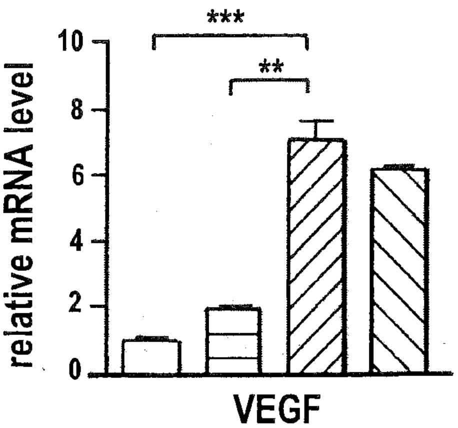


FIG. 6C

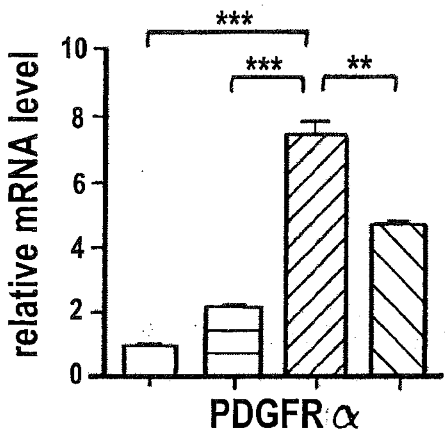


FIG. 6D

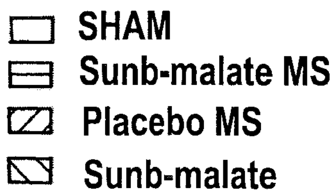


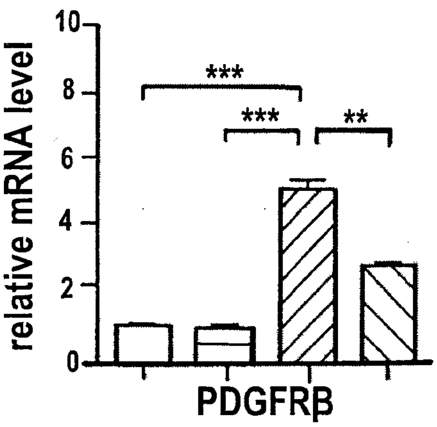


FIG. 6E

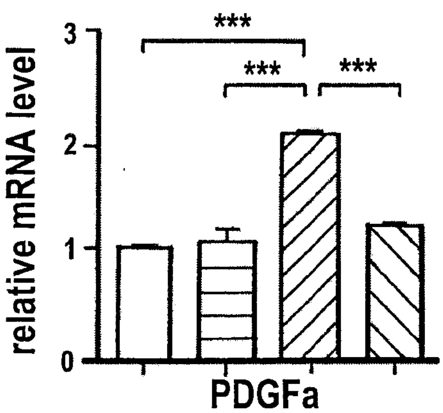


FIG. 6F

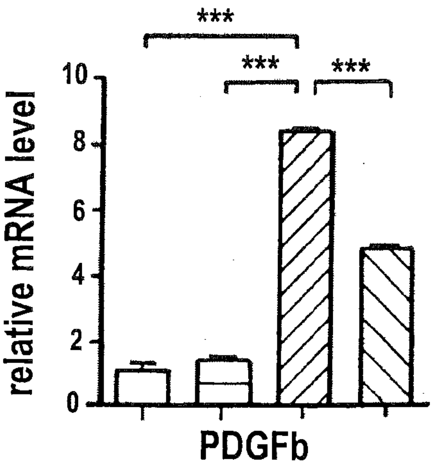


FIG. 6G

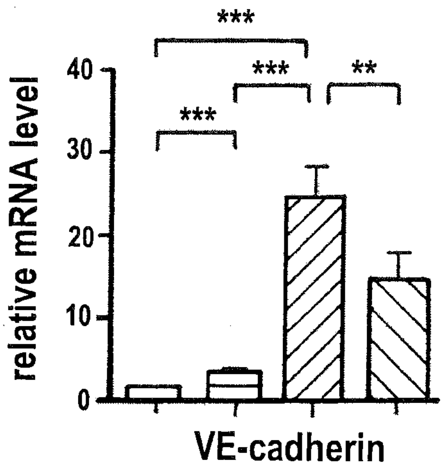


FIG. 6H

SHAM

Sunb-malate MS

Placebo MS

Sunb-malate

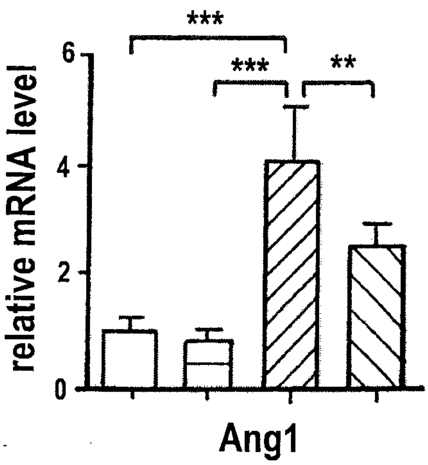


FIG. 6I

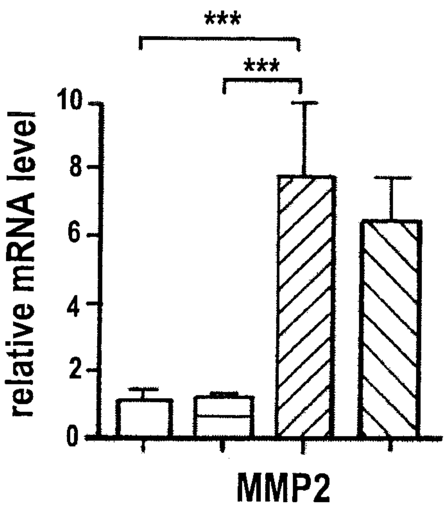


FIG. 6J

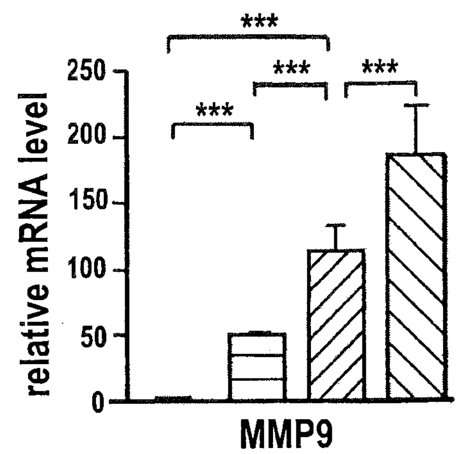


FIG. 6K

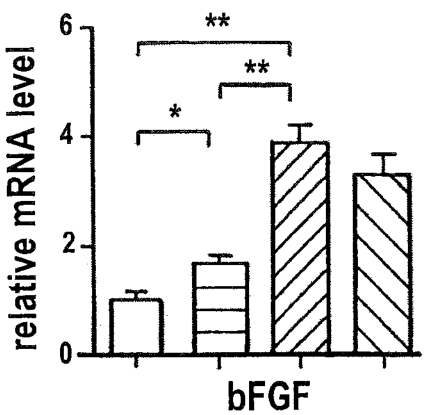


FIG. 6L

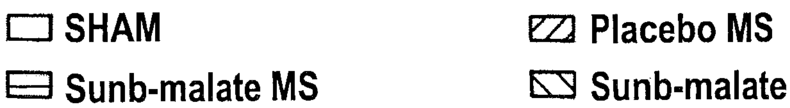


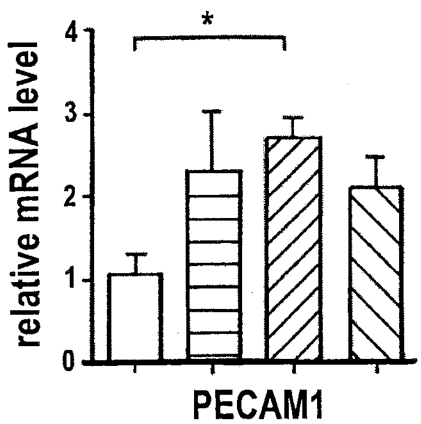


FIG. 6M

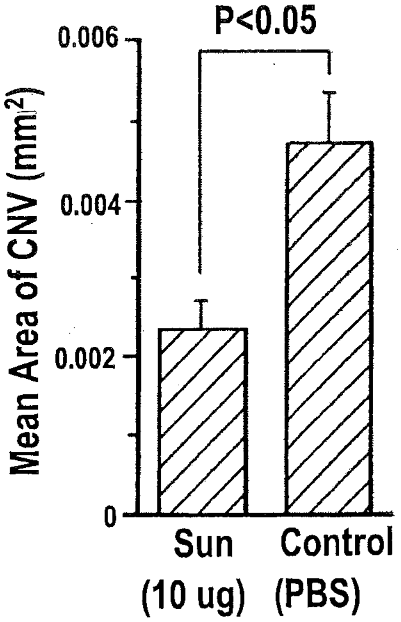


*FIG. 7A*

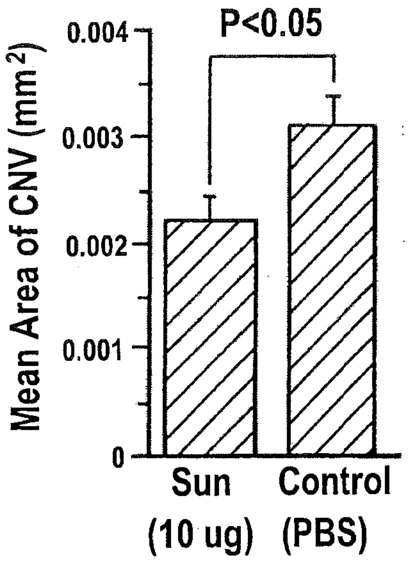


*FIG. 7B*

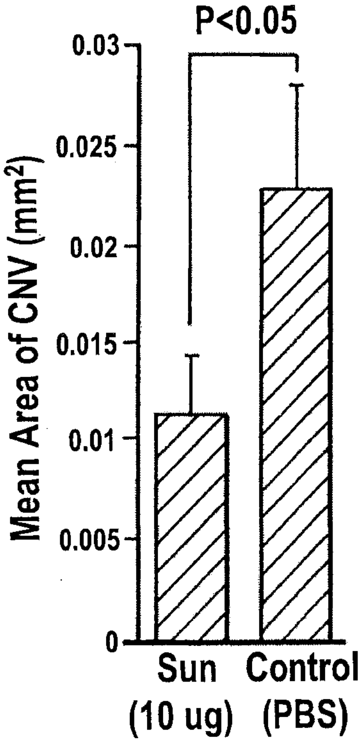


*FIG. 7C*

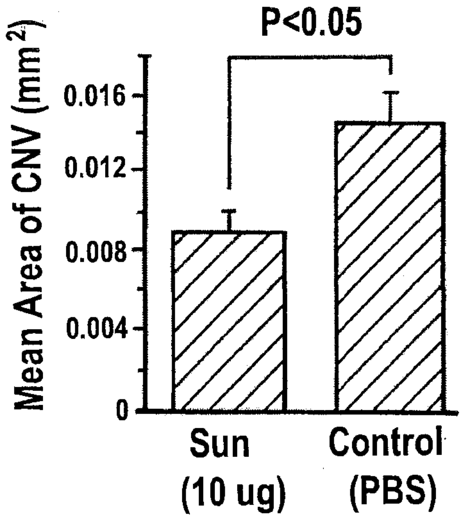


*FIG. 7D*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62092118
- US 62139306
- WO 2013177367 A
- GB 929406 B
- GB 929401 B
- US 3266987 A
- US 4794000 A
- US 4460563 A
- US 5019400 A, Gombotz
- US 4997652 A
- US 20100124565 A


### Non-patent literature cited in the description


- **LI et al.** *Int. J. Pharm.,* 2008, vol. 363, 26-39
- **FUCHS et al.** *Int. J. Pharm.,* 2014, vol. 482, 68-74
- **ZHAO et al.** *J. Chinese Pharm. Sci.,* 2014, vol. 23, 1003-1057
- **HERRERO-VANRELL et al.** *Progress in Retinal and Eye Res.,* 2014, vol. 42, 27-43
- **RAMAZANI et al.** *European Journal of Pharmaceutics and Biopharmaceutics,* 2015, vol. 95, 368-377
- Remington's Pharmaceutical Sciences. Lippincott Williams & Wilkins, 2000, 704
- **BRESSLER et al.** *JAMA,* 2004, vol. 291, 1900-1901
- **FERRIS et al.** *Arch. Ophthamol.,* 1984, vol. 102, 1640-2
- **D'AMICO D.** *J. N. Engl. J. Med.,* 1994, vol. 331, 95-106
- **AMPARO, F. et al.** *Investigative Ophthalmology & Visual Science,* 2013, vol. 54 (1), 537-544
- **CAKMAK, H. et al.** *Cutan Ocul Toxicol,* 2015, 1-7
- **PEREZ-SANTONJA, J.J. et al.** *Arch Soc Esp Oftalmol,* 2013, vol. 88 (12), 473-81
- **KO, B.Y. et al.** *Cornea,* 2013, vol. 32 (5), 689-95
- **GOVINDARAJAN, B. ; I.K. GIPSON.** *Exp Eye Res,* 2010, vol. 90 (6), 655-63
- **GAUDANA, R. et al.** *Pharm Res,* 2009, vol. 26 (5), 1197-216
- **MAKADIA, H.K ; S.J. SIEGEL.** *Polymers (Basel),* 2011, vol. 3 (3), 1377-1397
- **SHIVE, M.S ; J.M. ANDERSON.** *Adv Drug Deliv Rev,* 1997, vol. 28 (1), 5-24
- **TOBE, T. et al.** *Am. J. Pathol.,* 1998, vol. 135 (5), 1641-1646